(19) Europäisches Patentamt
European Patent Office
Office européen des brevets

(11) EP 2 874 737 B1

(12) EUROPÄISCHE PATENTSCHRIFT

(45) Veröffentlichungstag und Bekanntmachung des Hinweises auf die Patenterteilung:
**07.11.2018 Patentblatt 2018/45**

(21) Anmeldenummer: **13739612.3**

(22) Anmeldetag: **16.07.2013**

(51) Int Cl.:
***B01F 17/00*** *(2006.01)* ***C07C 309/17*** *(2006.01)*
***C11D 1/12*** *(2006.01)*

(86) Internationale Anmeldenummer:
**PCT/EP2013/002112**

(87) Internationale Veröffentlichungsnummer:
**WO 2014/012661 (23.01.2014 Gazette 2014/04)**

(54) **FLUORTENSIDE**

FLUOROSURFACTANTS

TENSIOACTIFS FLUORÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.07.2012 EP 12005257**

(43) Veröffentlichungstag der Anmeldung:
**27.05.2015 Patentblatt 2015/22**

(73) Patentinhaber: **Merck Patent GmbH**
**64293 Darmstadt (DE)**

(72) Erfinder:
- **FRIEDRICH, Reiner**
  **64342 Seeheim-Jugenheim (DE)**
- **JONSCHKER, Gerhard**
  **64646 Heppenheim (DE)**
- **SCHOOREN, Fanny**
  **64372 Ober-Ramstadt (DE)**
- **DEPNER, Christian**
  **55288 Gabsheim (DE)**
- **SCHELLENBERGER, Steffen**
  **64293 Darmstadt (DE)**

(56) Entgegenhaltungen:
**EP-A1- 0 284 331 EP-A1- 1 762 566**
**EP-A2- 0 285 160 WO-A1-02/03958**
**WO-A1-2009/094344 WO-A1-2010/149262**
**WO-A2-2006/116222 WO-A2-2010/002623**
**US-A1- 2009 324 834**

- **DATABASE CA [Online] CHEMICAL ABSTRACTS SERVICE, COLUMBUS, OHIO, US; OKURA, MASAHIRO ET AL: "Fluorine-containing organic peroxides, radical polymerization initiators, and manufacture of fluoropolymers with good heat stability and suppressed whitening, discoloration, and expansion", XP002714399, gefunden im STN Database accession no. 2010:1127580 -& JP 2010 195937 A (ASAHI GLASS CO., LTD., JAPAN) 9. September 2010 (2010-09-09)**
- **DATABASE CA [Online] CHEMICAL ABSTRACTS SERVICE, COLUMBUS, OHIO, US; IVANOVA, T. L. ET AL: "Hydroxyethylated .alpha.,.alpha.-dihydroperfluoroalcohols for preparation of nonfreezing polyacrylates", XP002714400, gefunden im STN Database accession no. 1978:547393 -& SU 618 367 A1 (USSR) 5. August 1978 (1978-08-05)**
- **DATABASE CA [Online] CHEMICAL ABSTRACTS SERVICE, COLUMBUS, OHIO, US; NAGAI, TAKAFUMI ET AL: "Perfluoropolyether-containing amphiphilic compounds and their uses", XP002714401, gefunden im STN Database accession no. 2004:52783 in der Anmeldung erwähnt -& JP 2004 018394 A (DAIKIN INDUSTRIES, LTD., JAPAN) 22. Januar 2004 (2004-01-22)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 874 737 B1

## Beschreibung

Gegenstand der vorliegenden Erfindung sind neue Verbindungen mit fluorierten Endgruppen, deren Verwendung als oberflächenaktive Substanzen und Mittel enthaltend diese Verbindungen.

Fluortenside, deren statische Oberflächenspannung sehr niedrig ist (16-18 mN/m), sind in verschiedensten Anwendungen einsetzbar und tragen z.B. zur verbesserten Benetzung von Oberflächen bei. So werden sie z.B. als Grenzflächenvermittler bzw. Emulgator oder Viskositätsminderer in Farben, Lacken oder Klebstoffen verwendet.

In WO 03/010128 werden Perfluoralkyl-substituierte Amine, Säuren, Aminosäuren und Thioethersäuren beschrieben, die eine $C_{3-20}$-Perfluoralkyl-Gruppe aufweisen. Aus JP-A-2001/133984 sind oberflächenaktive Verbindungen mit Perfluoralkoxy-Ketten bekannt, die sich zum Einsatz in Antireflex-Beschichtungen eignen. Aus JP-A-09/111286 ist die Verwendung von Perfluorpolyethertensiden in Emulsionen bekannt. In WO 2006/072401 und WO 2010/003567 werden oberflächenaktive Verbindungen mit Trifluormethoxygruppen beschrieben. Verbindungen mit speziellen Fluoralkylgruppen sind in US 7,635,789, US 2008/0093582, JP 2004-18394 und WO 2010/002623 beschrieben. Außerdem ist die Verbindung $CF_3$-$CF_2$-$CF_2$-O-$CH_2$-$CH_2$-OH bekannt (CAS 1313023-37-8) und in J. of Supercritical Fluids 55 (2010) 802-816 sind theoretische Untersuchungen an Mischungen aus $CO_2$ und Perfluoralkyltensiden in wässrigen Lösungen beschrieben. EP 1 762 566 A1 beschreibt Verbindungen mit jeweils nur einer Perfluoralkylgruppe.

Spezielle Anwendungen von Sulfosuccinaten und/oder Sulfotricarballylaten mit verschiedenen fluorierten Seitenketten sind in US 4,968,599 und US 4,988,610 sowie US 6, 890, 608 beschrieben und in A.R. Pitt et al., Colloids and Surfaces A: Physicochemical and Engineering Aspects, 1996, 114, 321-335; A.R. Pitt, Progr. Colloid Polym. Sci, 1997, 103, 307-317 und Z.-T. Liu et al., Ind. Eng. Chem. Res. 2007, 46, 22-28. Weitere Fluortenside, insbesondere Succinate und Tricarballylate mit fluorierten Alkylgruppen, sind in WO 2009/149807, WO 2010/003567, WO 2010/149262, WO 2011/082770 und WO 2012/084118 beschrieben.

Weiterhin besteht Bedarf nach alternativen oberflächenaktiven Substanzen, vorzugsweise mit einem, den klassischen Fluortensiden, vergleichbaren Eigenschaftsprofil, die vorzugsweise beim Abbau nicht zu langkettigen persistenten Verbindungen abbauen oder vorzugsweise in geringerer Dosierung genauso effektiv sind wie herkömmliche Fluortenside.

Es wurden nun neue Verbindungen gefunden, die als oberflächenaktive Substanzen geeignet sind und bevorzugt einen oder mehrere der o.g. Nachteile nicht aufweisen.

Ein erster Gegenstand der vorliegenden Erfindung sind Verbindungen der Formeln (I), (II) oder (III) gemäß Anspruch 1

$$(R_f\text{-}A_a\text{-}C_c)_n\text{-}(Spacer)_m\text{-}X_o \qquad (I)$$

wobei

für Rf = $CF_3$-$CF_2$-$CF_2$-O-CF($CF_3$)-$CH_2$-
A = -O-$CH_2$-CHR'-,
R' = H oder C1-C4 Alkyl,
C = C1-C4 Alkylen, O- oder -OCO-,
Spacer = eine lineare oder verzweigte, gegebenenfalls mit Heteroatomen versehene, Kohlenwasserstoffeinheit,
X eine anionische, kationische, nicht-ionische oder amphotere Gruppe ist, a = 1,
n = 2 oder 3,
c = 1,
m = 1,
o = 1 oder 2,und
für Rf = $CF_3$-$CF_2$-$CF_2$-O-CF($CF_3$)-CO-,
C = -NR"- mit R" = H, Alkyl, oder -$(CH_2CHR''')_{m'}$-R"" mit m' = eine ganze Zahl aus dem Bereich von 1 bis 100 und R"'= H oder C1-C4-Alkyl und R"" = Alkyl, wobei ein oder mehrere nicht benachbarte C-Atome durch O ersetzt sein können
Spacer = lineares oder verzweigtes Alkylen,
X eine anionische, kationische, nicht-ionische oder amphotere Gruppe ist, a = 0,
n = 2 oder 3,
c = 1,
m = 1, und
o = 1 oder 2, und o = 0 wenn R" = -$(CH_2CHR''')_m$-R"";

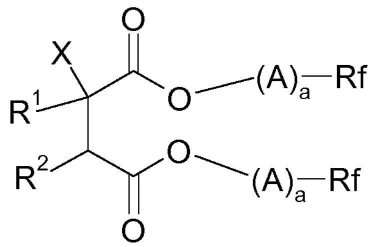

(II)

wobei

Rf = $CF_3$-$CF_2$-$CF_2$-O-$CF(CF_3)$-$CH_2$--,
A = -O-$CH_2$-CHR'-,
R' = H, $CH_3$, $C_2H_5$
a = 1 ist
X eine anionische, kationische, nicht-ionische oder amphotere Gruppe ist,
$R^1$ = Wasserstoff oder -$CH_2$-COO-$(A)_a$-Rf,
$R^2$ = Wasserstoff, -$CH_2$-COO-$(A)_a$-Rf oder X, ist;

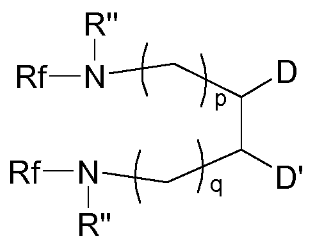

(III)

wobei

Rf = $CF_3$-$CF_2$-$CF_2$-O-$CF(CF_3)$-CO- oder = Rf' mit
Rf' = $CF_3$-$CF_2$-$CF_2$-O-$CF(CF_3)$-$CH_2$-O-$CH_2$-CHR'-,
p = 1-10,
q = 1-10 und
R' und R" die für Formel (I) beschriebenen Bedeutungen haben,
D und/oder D' = H oder X, wobei X eine anionische, kationische, nicht-ionische oder amphotere Gruppe ist und mindestens eine Gruppe gleich X ist, wenn R" = H oder Alkyl.

Bei den Kohlenwasserstoff-Einheiten des Spacers der Verbindungen der Formel (I) kann es sich um aliphatische oder aromatische, gegebenenfalls mit Heteroatomen versehene Einheiten handeln. Vorzugsweise ist die Gruppierung $(R_f\text{-}A_a\text{-}C_c)_n$ in den oberflächenaktiven Verbindungen dabei an eine gesättigte, verzweigte oder unverzweigte Kohlenwasserstoff-Einheit, bevorzugt an eine gesättigte, verzweigte oder unverzweigte Alkylengruppe, wobei ein oder mehrere nicht benachbarte C-Atome durch O oder N, bevorzugt O, ersetzt sein können, gebunden. In einer Erfindungsvariante wird als bevorzugte Heteroatome enthaltende Kohlenwasserstoffeinheit eine Polyethylen- oder Polypropylenglykoleinheit verwendet.
Die Gruppierung $(R_f\text{-}A_a\text{-}C_c)_n$ kommt in der oberflächenaktiven Verbindung zweimal oder dreimal vor.
R"' ist gleich H oder C1-C4 Alkyl. R"" ist bevorzugt gleich Alkyl, wobei ein oder mehrere nicht benachbarte C-Atome durch O ersetzt sein können.
Bevorzugt sind Verbindungen der Formel (I) in denen:

Rf = $CF_3$-$CF_2$-$CF_2$-O-$CF(CF_3)$-$CH_2$-,
A = -O-$CH_2$-CHR'-,
R' = H, $CH_3$ oder $C_2H_5$, insbesondere H,
C = C1-C2, Alkylen, O oder-OCO-
Spacer = eine lineare oder verzweigte, gegebenenfalls mit Heteroatomen versehene, Kohlenwasserstofffeinheit,
X eine anionische, kationische, nicht-ionische oder amphotere Gruppe ist,

a = 1,
n = 2 oder 3,
c = 1,
m = 1,
o = 1 oder 2 ist.

Weitere bevorzugte Verbindungen der Formel (I) sind solche, in denen:

Rf = $CF_3$-$CF_2$-$CF_2$-O-CF($CF_3$)-CO-,
C = -NR"- mit R" = H, Alkyl, oder -($CH_2$CHR"')$_{m'}$-R"" mit m' = eine ganze Zahl aus dem Bereich von 1 bis 100 und R"' = H oder C1-C4-Alkyl und R"" = Alkyl, wobei ein oder mehrere nicht benachbarte C-Atome durch O ersetzt sein können,
Spacer = lineares oder verzweigtes Alkylen,
X eine anionische, kationische, nicht-ionische oder amphotere Gruppe ist,
a = 0,
n = 2,
c = 1,
m = 1,
o = 1 oder 2 ist und o = 0 wenn R" = -($CH_2$CHR"')$_{m'}$-R"".
Bevorzugt ist R" gleich H oder C1-C4 Alkyl, bevorzugt C1-C2 Alkyl.

In einer Erfindungsvariante ist R" bevorzugt gleich -($CH_2$CHR"')$_{m'}$-R"", bevorzugt mit m' = 1 bis 30, insbesondere 1-15, und R"' = H oder $CH_3$ und R"" = Alkyl, wobei ein oder mehrere nicht benachbarte C-Atome durch O ersetzt sind.
Insbesondere bevorzugt sind Verbindungen der Formel (II):

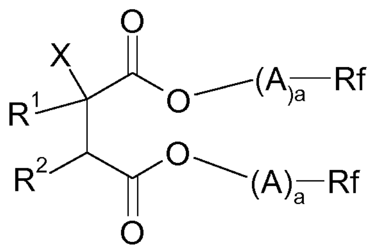

(II)

wobei

Rf = $CF_3$-$CF_2$-$CF_2$-O-CF($CF_3$)-$CH_2$-- ist,
A = -O-$CH_2$-CHR'-,
R' = H, $CH_3$, $C_2H_5$
X eine anionische, kationische, nicht-ionische oder amphotere Gruppe ist,
$R^1$ = Wasserstoff oder -$CH_2$-COO-(A)$_a$-Rf,
$R^2$ = Wasserstoff, -$CH_2$-COO-(A)$_a$-Rf oder X
a = 1.

Eine weitere bevorzugte Variante der Erfindung sind Verbindungen der Formel (III):

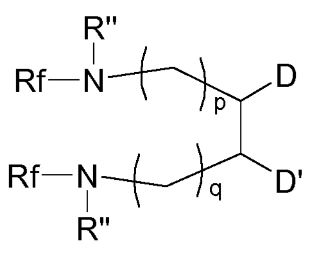

(III)

wobei

Rf = $CF_3$-$CF_2$-$CF_2$-O-CF($CF_3$)-CO- oder = Rf' mit Rf' = $CF_3$-$CF_2$-$CF_2$-O-CF($CF_3$)-$CH_2$-O-$CH_2$-CHR'-,
p = 1-10,
q = 1-10 und
R' und R" die für Formel (I) beschriebenen bevorzugten Bedeutungen haben.

D und/oder D' sind gleich H oder X, wobei mindestens eine Gruppe gleich X sein muss, wenn R" = H oder Alkyl. X ist eine anionische, kationische, nicht-ionische oder amphotere Gruppe. p und q sind bevorzugt unabhängig voneinander 1-4, insbesondere 1-2. Bevorzugt sind p und q gleich. Bevorzugt ist Rf in Formel (III) gleich $CF_3$-$CF_2$-$CF_2$-O-CF($CF_3$)-CO-.

In einer Variante der Erfindung ist R"" in der Formel (III) gleich der im Folgenden als Beispiel einer hydrophilen Gruppe X beschriebenen nicht-ionischen Gruppe -Y-($CH_2$-$CH_2$-O)$_v$-$R^4$ mit Y = O, $R^4$ = H oder Alkyl, bevorzugt H oder $CH_3$, und v = 1-100, bevorzugt 1-20, insbesondere 1-15. In den erfindungsgemäßen Verbindungen ist X eine anionische, kationische, nicht-ionische oder amphotere Gruppe.

Eine bevorzugte anionische Gruppe X kann ausgewählt sein aus $-COO^-$, - $SO_3^-$, $-OSO_3^-$, $-PO3^{2-}$, $-OPO3^{2-}$, $-(OCH_2CH_2)_s-O-(CH_2)_t-COO^-$, $-(OCH_2CH_2)_s-O-(CH_2)_t-SO_3^-$, $-(OCH_2CH_2)_s-O-(CH_2)_t-OSO3^-$, $-(OCH_2CH_2)_s-O-(CH_2)_t-PO_3^{2-}$, $-(OCH_2CH_2)_s-O-(CH_2)_t-OPO_3^{2-}$ oder aus den Formeln A bis C,

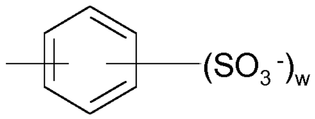

A

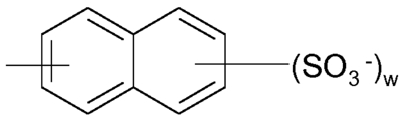

B

oder

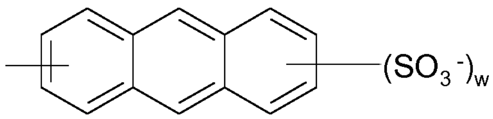

C

wobei s steht für eine ganze Zahl aus dem Bereich von 1 bis 1000, t steht für eine ganze Zahl ausgewählt aus 1, 2, 3 oder 4 und w steht für eine ganze Zahl ausgewählt aus 1, 2 oder 3.

Zu den bevorzugten anionischen Gruppen gehören dabei insbesondere - $COO^-$, $-SO_3^-$, $-OSO_3^-$, $-PO_3^{2-}$, $-OPO_3^{2-}$, die Teilformel A, sowie $-(OCH_2CH_2)_s-O-(CH_2)_t-COO^-$, $-(OCH_2CH_2)_s-O-(CH_2)_tSO_3^-$ und - $(OCH_2CH_2)_s-O-(CH_2)_t-OSO_3^-$, wobei jede einzelne dieser Gruppen für sich genommen bevorzugt sein kann.

Zu den ganz besonders bevorzugten anionischen Gruppen gehören dabei $-SO3^-$, $-OSO_3^-$, $-PO_3^{2-}$, oder $OPO_3^{2-}$. Insbesondere eine Sulfonatgruppe $-SO_3^-$ ist bevorzugt.

Bevorzugtes Gegenion für anionische Gruppen X ist ein einwertiges Kation, insbesondere $H^+$, ein Alkalimetall-Kation oder $NR_4^+$, wobei R = H oder C1 - C6-Alkyl ist und alle R gleich oder verschieden sein können. Insbesondere bevorzugt sind $Na^+$, $K^+$, $Li^+$ und $NH_4^+$, besonders bevorzugt $Na^+$.

Eine bevorzugte kationische Gruppe X kann ausgewählt sein aus $-NR^1R^2R^{3+}Z^-$, $-PR^1R^2R^{3+}Z^-$,

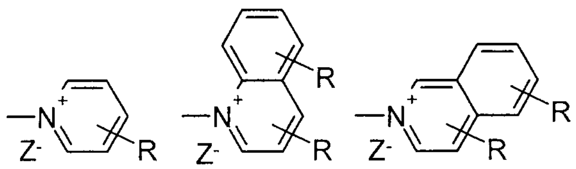

wobei R steht für H oder $C_{1-4}$-Alkyl in beliebiger Position, $Z^-$ steht für $Cl^-$, $Br^-$, $I^-$, $CH_3SO_3^-$, $CF_3SO_3^-$, $CH_3PhSO_3^-$, $PhSO_3^-$ $R^1$, $R^2$ und $R^3$ jeweils unabhängig voneinander stehen für H, $C_{1-30}$-Alkyl, Ar oder -$CH_2$Ar und
Ar steht für einen unsubstituierten oder ein- oder mehrfach substituierten aromatischen Ring oder kondensierte Ringsysteme mit 6 bis 18 C-Atomen, worin auch ein oder zwei CH-Gruppen durch N ersetzt sein können.

Zu den bevorzugten kationischen Gruppen gehören dabei insbesondere $-NR^1R^2R^{3+}$ $Z^-$ und

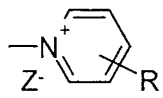

wobei jede einzelne dieser Gruppen für sich genommen bevorzugt sein kann.

Eine bevorzugte nicht-ionische Gruppe kann ausgewählt sein aus: lineares oder verzweigtes Alkyl, wobei ein oder mehrere nicht benachbarte C-Atome durch O, S, und/oder N ersetzt sind, -OH, -SH, -O-(Glycosid)$_{o'}$, -S-(Glycosid)$_{o'}$, $-OCH_2-CHOH-CH_2-OH$, $-OCH_2Ar(-NCO)_{p'}$, $-OAr(-NCO)_{p'}$, Aminoxid,

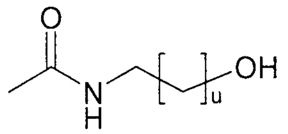

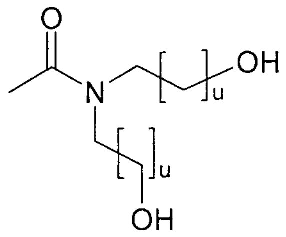

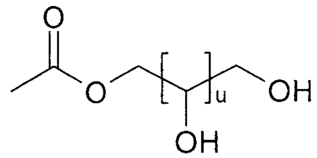

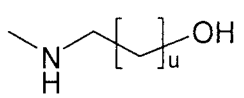

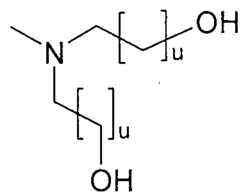

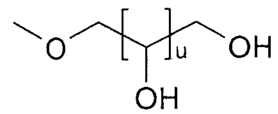

u steht für eine ganze Zahl aus dem Bereich von 1 bis 6, bevorzugt 1 bis 4 o' steht für eine ganze Zahl aus dem Bereich von 1 bis 10,

p' steht für 1 oder 2,

Ar steht für einen unsubstituierten, ein- oder mehrfach substituierten aromatischen Ring oder kondensierte Ringsysteme mit 6 bis 18 C-Atomen, worin auch ein oder zwei CH-Gruppen durch C=O ersetzt sein können und, Glycosid steht für ein verethertes Kohlenhydrat, vorzugsweise für ein mono- di-, tri- oder oligo-Glucosid.

Zu den bevorzugten nicht-ionischen Gruppen gehören dabei insbesondere lineares oder verzweigtes Alkyl, wobei ein oder mehrere nicht benachbarte C-Atome durch O, S und/oder N ersetzt sind, -OH und -O-(Glycosid)$_{o'}$.

Wenn X = Alkyl, wobei ein oder mehrere nicht benachbarte C-Atome durch O, S, und/oder N ersetzt sind, dann ist es bevorzugt gleich $R^4$-(B-A)$_{m''}$- mit $R^4$ = H oder C1-4-Alkyl insbesondere H oder $CH_3$, A = lineares oder verzweigtes Alkylen, bevorzugt mit 1 bis 10 Kohlenstoffatomen, insbesondere mit 1 bis 4 Kohlenstoffatomen, B = O oder S, bevorzugt O, und m" = eine ganze Zahl bevorzugt aus dem Bereich von 1 bis 100, besonders bevorzugt 1 bis 30.

Insbesondere bevorzugt als nicht-ionische Gruppe X ist die Gruppe $R^4$-(O-$CH_2CHR^5$)$_{m''}$- mit m" = eine ganze Zahl aus dem Bereich von 1 bis 100, bevorzugt 1 bis 30, insbesondere 1-15, und $R^4$ und $R^5$ = H oder C1-4-Alkyl, insbesondere H oder $CH_3$. Insbesondere bevorzugt ist $R^4$-(B-A)$_{m''}$-eine Polyethylen- oder Polypropylenglykoleinheit.

Insbesondere bevorzugt als nicht-ionische Gruppe X ist die Gruppe $-CH(OH)-CH_2-NH$-Sach mit Sach = verschiedene Zucker und die Gruppe - Y-($CH_2$-$CH_2$-O)$_v$-$R^4$ mit Y = S, O oder NH, bevorzugt O, $R^4$ = H oder Alkyl, bevorzugt H oder $CH_3$, und v = 1-100, bevorzugt 1-20, insbesondere 1-15.

Eine bevorzugte amphotere Gruppe kann ausgewählt sein aus den funktionellen Gruppen der Acetyldiamine, der N-Alkylaminosäuren, der N-Alkylaminosulfonsäuren, der Betaine, der Sulfobetaine, bzw. entsprechender Derivate, insbesondere ausgewählt aus, wobei M steht für H oder ein Alkalimetall-Ion, vorzugsweise $Li^+$, $Na^+$ oder $K^+$:

| |
|---|
| 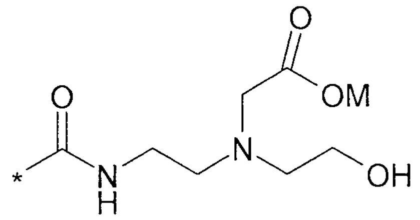 |
| 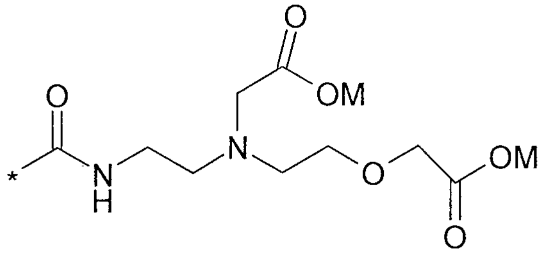 |
| 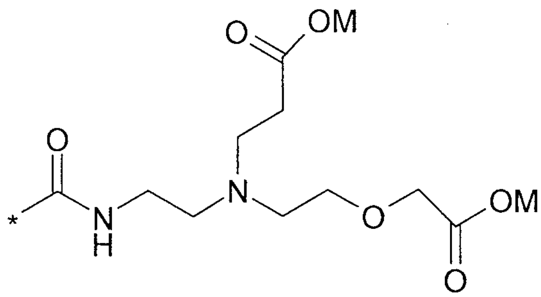 |
| $-NH-CH_2-COOM$; $-NH-CH_2-CH_2-COOM$ |
| 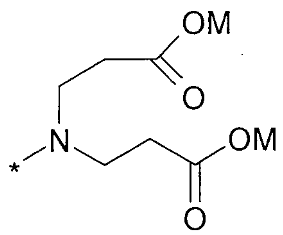 |
| $-[(C(=O)-NH-(CH_2)_{(1-8)}]_{(0\ oder\ 1)}-N^+R^1R^2-CH_2-COO^-$, wobei $R^1$ und $R^2$ jeweils unabhängig voneinander stehen für einen C1-8-Alkylrest, vorzugsweise Methyl oder Ethyl |
| $-C(=O)-NH-(CH_2)_{1-3}-N^+R^1R^2-CH_2-CH(OH)-CH_2-(O)_{(0\ oder\ 1)}-(S\ oder\ P)O_3^-$, wobei $R^1$ und $R^2$ jeweils unabhängig voneinander stehen für einen C1-8-Alkylrest, vorzugsweise Methyl oder Ethyl |

Besonders bevorzugte erfindungsgemäße Verbindungen sind solche, die als hydrophile Gruppe X eine der bevorzugten anionische Gruppen, der bevorzugten nicht-ionischen Gruppen oder der bevorzugten zwitterionischen Gruppen enthalten.

Insbesondere bevorzugt sind Verbindungen, die die Gruppen $-SO_3^-$, $-OSO_3^-$, $-PO_3^{2-}$ oder $OPO_3^{2-}$, Polyethylen- oder Polypropylenglykole, $-CH(OH)-CH_2-NH-Sach$, $-Y-(CH_2-CH_2-O)_v-R^4$, Betaine, oder Sulfobetaine enthalten. Bevorzugte Gegenionen sind hierbei $Na^+$, $K^+$ und $NH_4^+$, insbesondere $Na^+$. Insbesondere bevorzugt sind: $-SO_3^-$, Polyethylen- oder Polypropylenglykole, Sulfobetaine, die Gruppe $-CH(OH)-CH_2-NH-Sach$ und die Gruppe $-Y-(CH_2-CH_2-O)_v-R^4$. Hierbei ist Sach = verschiedene Zucker und Y = S, O oder NH, bevorzugt O, $R^4$ = H oder Alkyl, bevorzugt H oder $CH_3$, und v = 1-100, bevorzugt 1-20, insbesondere 1-15. Besonders vorteilhaft sind Verbindungen mit X = $-SO_3^-$.

Besonders vorteilhaft sind Verbindungen der Formel (I), in denen eine oder mehrere der Variablen die bevorzugten Bedeutungen haben. Besonders vorteilhaft sind Verbindungen der Formel (I), in denen alle genannten Variablen die bevorzugten Bedeutungen haben.

Insbesondere bevorzugt sind Verbindungen der Formeln (II) und (III) in denen eine oder mehrere der Variablen

die bevorzugten Bedeutungen haben. Besonders die Varianten der Formeln (II) bis (III) sind bevorzugt in der alle Variablen die bevorzugten Bedeutungen haben, vor Allem die besonders bevorzugten Bedeutungen. Besonders vorteilhaft sind Verbindungen der Formel (II), in denen alle genannten Variablen die bevorzugten Bedeutungen haben.

Bevorzugt sind vor allem Verbindungen der Formeln (II-a) (II-b), (II-c) und (II-d) in denen die Variablen die für die Formel (II) angegebenen Bedeutungen haben, insbesondere die bevorzugten Bedeutungen.

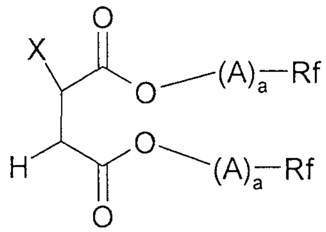

(II-a)

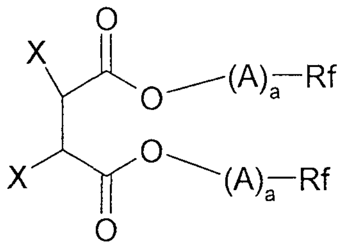

(II-b)

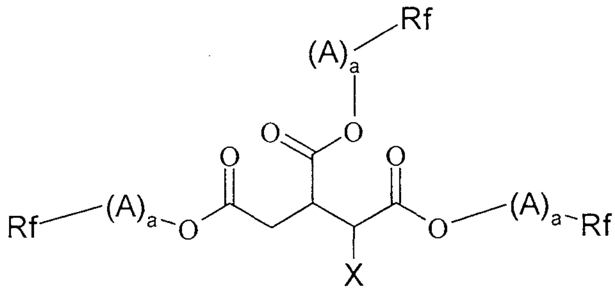

(II-c)

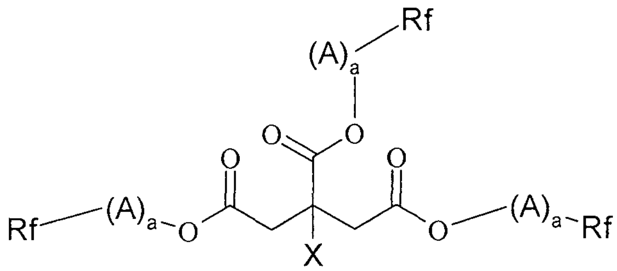

(II-d)

Beispiele besonders vorteilhafter Verbindungen sind die Succinate der Formeln (II-a/1), (II-a/2), (II-a/3) und (II-a/4) und die entsprechenden Tricarballylate, wobei R' die vorstehend beschriebene Bedeutung, insbesondere die bevorzugten Bedeutungen, hat:

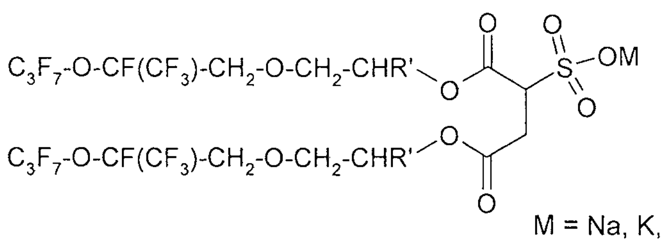

(II-a/1)

M = Na, K, $NH_4$

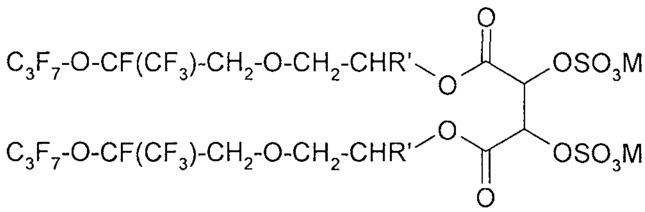

(II-a/2)

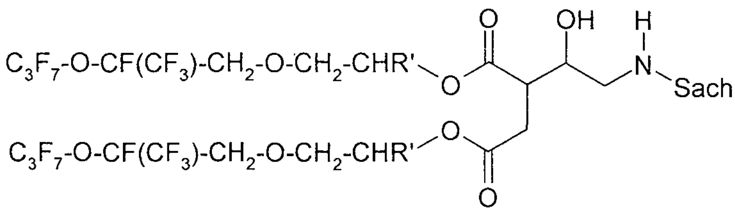

(II-a/3)

Sach = verschiedene Zucker

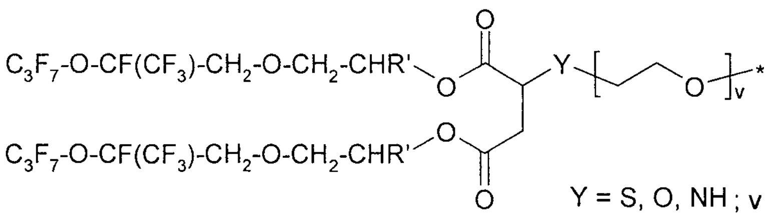

(II-a/4)

Y = S, O, NH ; v = 1-15

Bevorzugt sind auch Verbindungen der Formeln (III-1) bis (III-9), insbesondere der Formeln (III-6) bis (III-9), wobei p, q, R' und R" die gemäß Formel (III) beschriebenen Bedeutungen, insbesondere die bevorzugten Bedeutungen, haben:

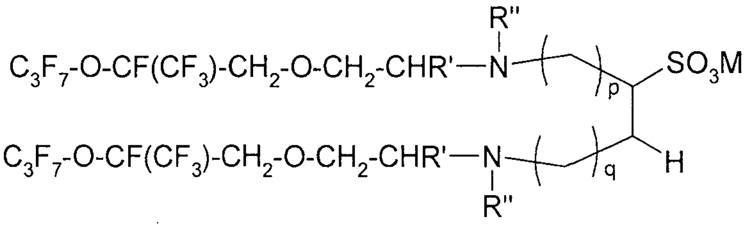

(III-1)

M = Na, K, NH4

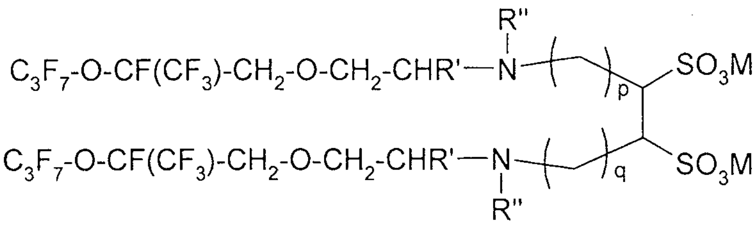

(III-2)

M = Na, K, NH4

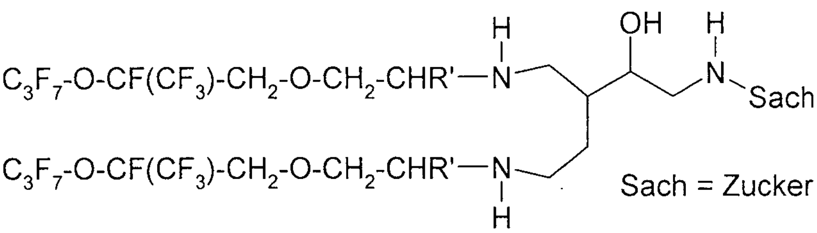

(III-3)

Sach = Zucker

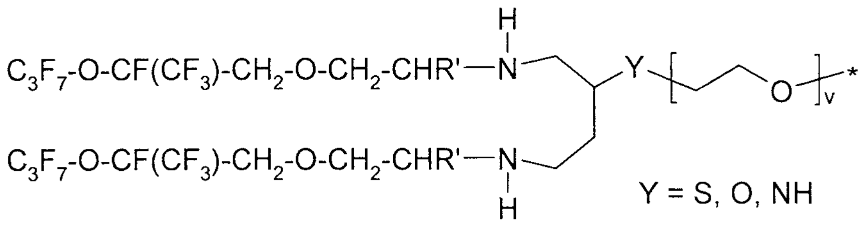

(III-4)

Y = S, O, NH

v = 1-100

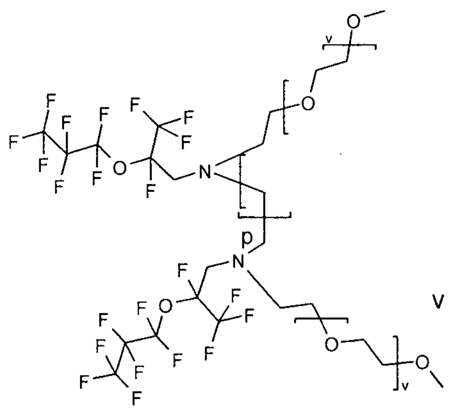

(III-5)

v = 1-100

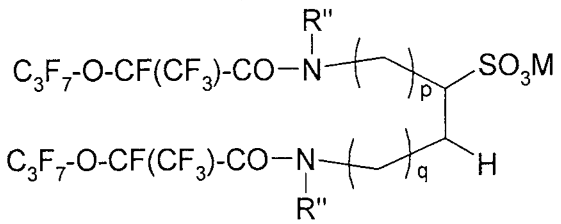

(III-6)

M = Na, K, NH4

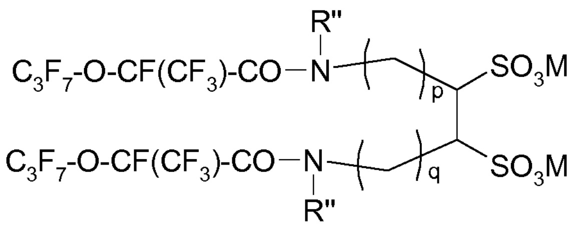

(III-7)

M = Na, K, NH4

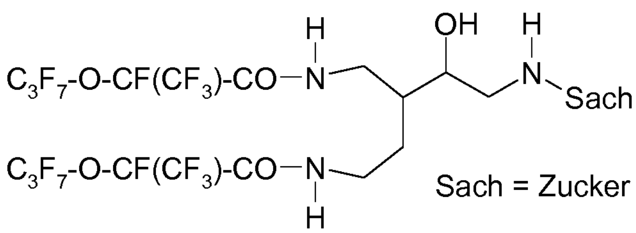

(III-8)

Sach = Zucker

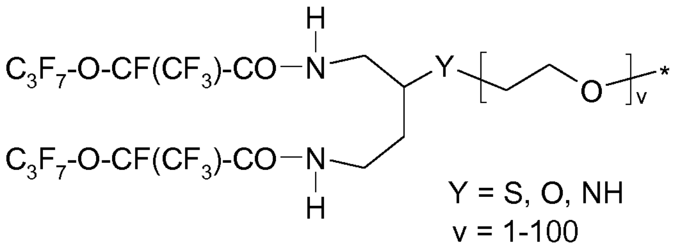

Y = S, O, NH
v = 1-100

(III-9)

Besonders vorteilhaft sind die bevorzugten Verbindungen der Formeln (II-a/1) und (II-a/2)). Bevorzugtes Gegenion ist hierbei $Na^+$.

Die erfindungsgemäßen Verbindungen der Formel (II) können bevorzugt durch Veresterung von Maleinsäure und Aconitsäure bzw. deren Anhydriden oder Säurechloriden mit einem oder mehreren Alkoholen der Formel (V), wobei Rf, A und a die für Formel (II) beschriebenen Bedeutungen bzw. bevorzugten Bedeutungen haben

$$Rf\text{-}(A)_a\text{-}OH \qquad (V)$$

und anschließender Addition an die Doppelbindung zur Einführung der Gruppierung X hergestellt werden.

Ein weiterer Gegenstand der Erfindung sind somit die entsprechenden Maleinsäure- und Aconitsäureester der Formeln (VI) bzw. (VII):

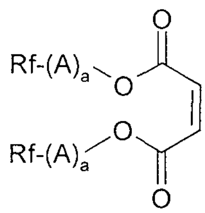

(VI)

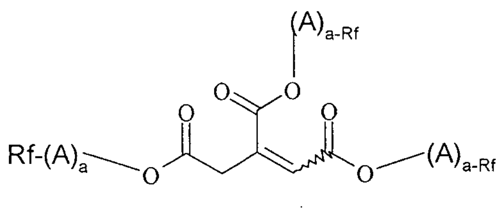

(VII)

Die Variablen haben in den Formeln (VI) und (VII) die für die Formel (II) beschriebenen Bedeutungen, insbesondere auch die bevorzugten Bedeutungen. Die Formel (VII) gibt das Vorliegen von Z/E-Doppelbindungs-Isomeren wieder.

Die erfindungsgemäßen Verbindungen können auch bevorzugt durch Veresterung von Hydroxybernsteinsäure und Zitronensäure mit einem oder mehreren Alkoholen der Formel (V) und anschließende Funktionalisierung der Hydroxygruppen zur Einführung der Gruppierung X hergestellt werden.

Die verwendeten Alkohole sind kommerziell erhältlich und/oder ihre Herstellung ausgehend von kommerziell erhältlichen Edukten ist dem Fachmann geläufig oder sie können in Analogie zu bekannten Syntheseverfahren hergestellt werden, z. B. durch Reduktion des Methyl Perfluor(2-methyl-3-oxahexanoats (z.B. mit $LiAlH_4$) und ggf. Kettenverlängerung mit Ethylencarbonat unter dem Fachmann bekannten Bedingungen.

Die Synthese erfindungsgemäßer Succinate bzw. Tricarballylate erfolgt bevorzugt in einer zweistufigen Synthese über die entsprechenden Maleate oder Hydroxysuccinate bzw. die entsprechenden Aconit- oder Zitronensäureester. Die folgenden Schemata zeigen dies beispielhaft:

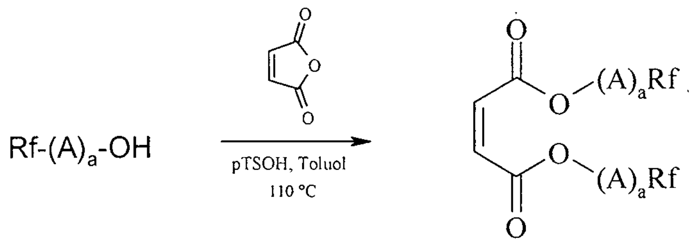


(V) (VI)

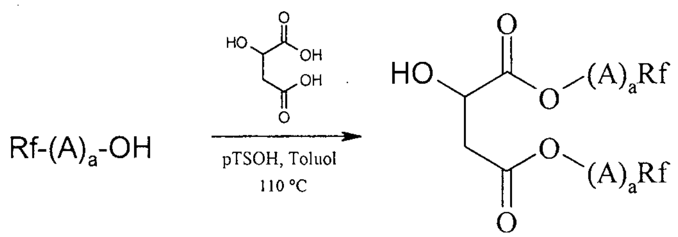


(V) (VIII)

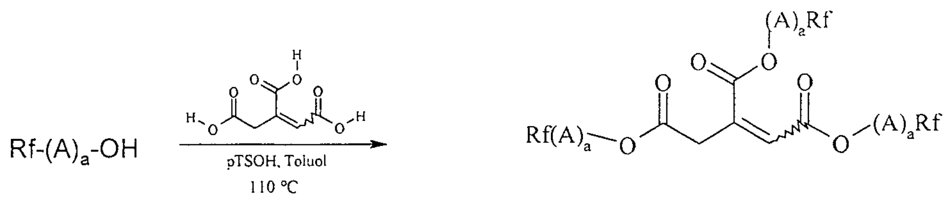


(V) (VII)

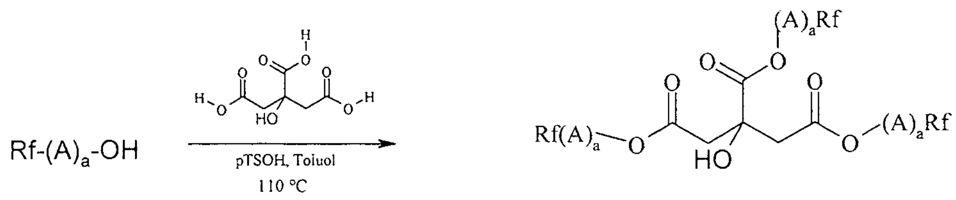


(V) (IX)

In einem zweiten Schritt erfolgt dann die Einführung der Gruppe X durch Addition an die Doppelbindung bzw. Derivatisierung der OH-Gruppe nach dem Fachmann geläufigen Methoden.

Das folgende Schema zeigt beispielhaft die Synthese der Sulfotricarballylate durch die Addition von Natriumhydrogensulfit, die unter dem Fachmann bekannten Bedingungen erfolgen kann:

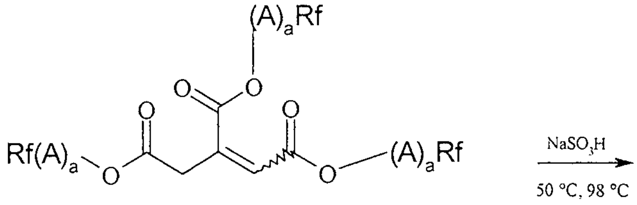


(VII)

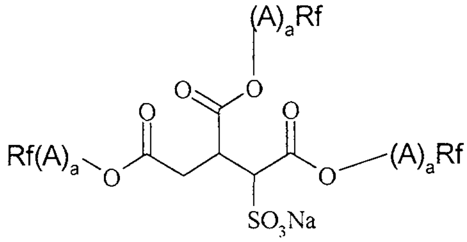

und/oder

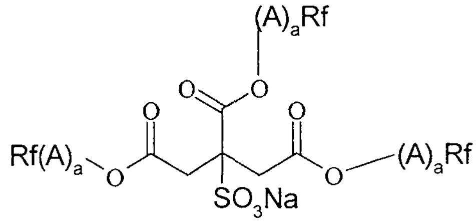

(II-c) (II-d)

Weitere Möglichkeiten zur Synthese von nichtionischen Tensiden aus Zitronen-/Aconitsäure sind in den folgenden Schemata beispielhaft aufgezeigt, wobei bevorzugt n = 1-30.

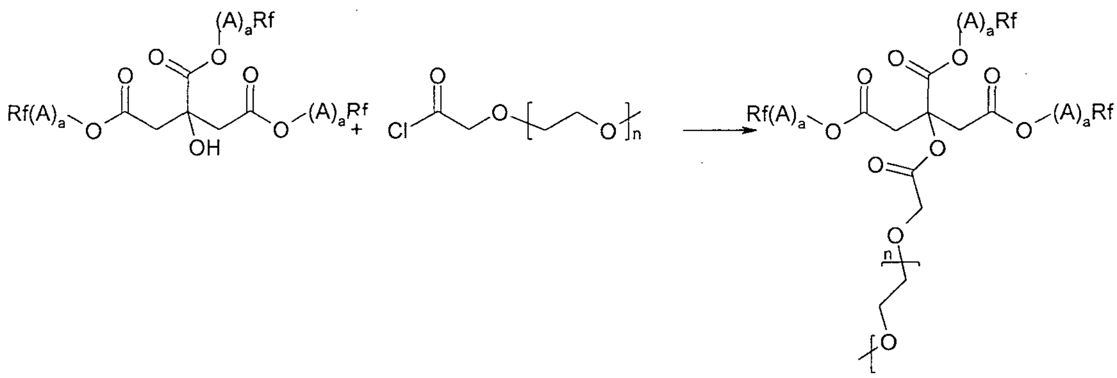

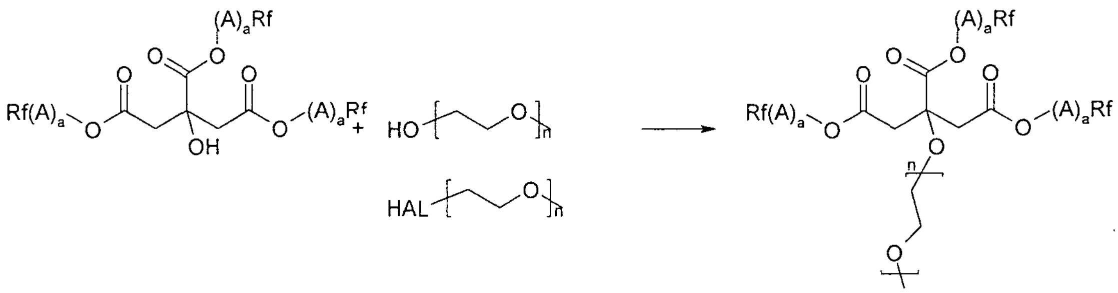

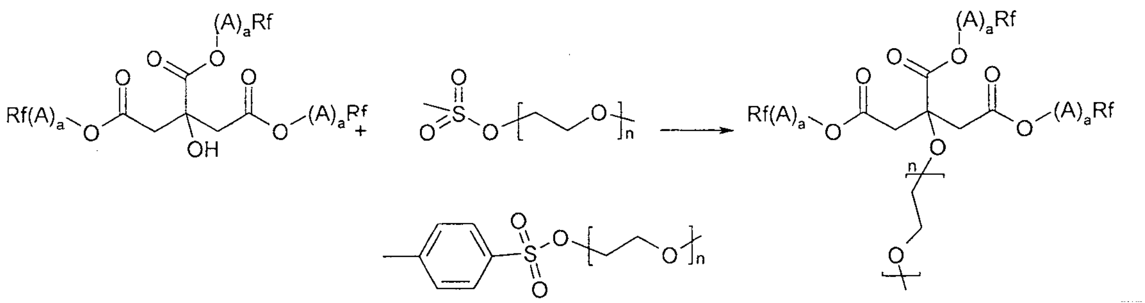

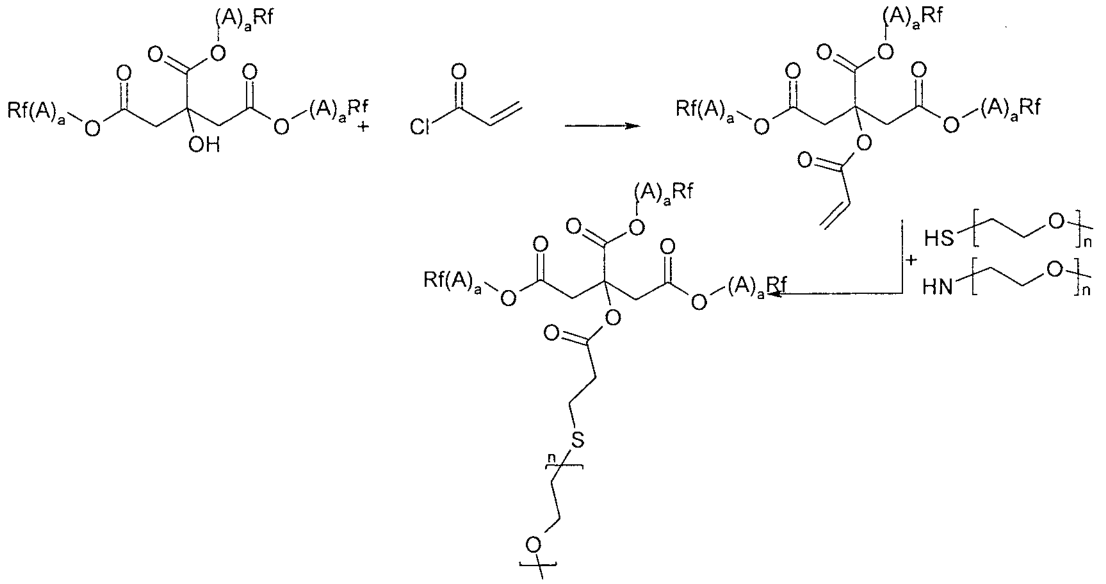

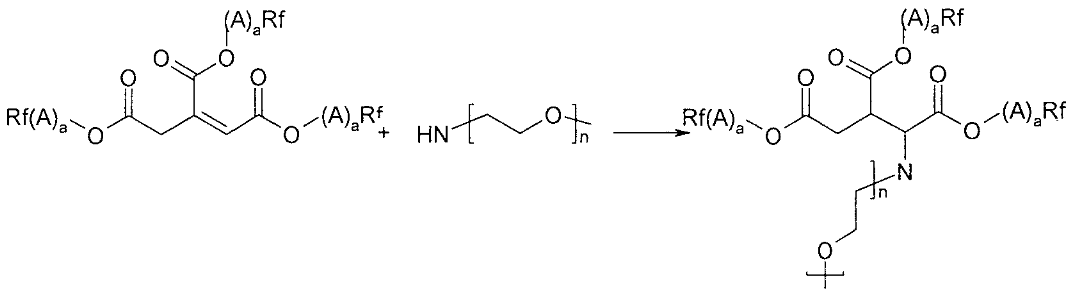

Die Herstellung weiterer erfindungsgemäßer Verbindungen der Formel(II) kann analog zu den oben gezeigten beispielhaften Reaktionen erfolgen. Die Herstellung weiterer erfindungsgemäßer Verbindungen der Formel (II) kann auch nach anderen dem Fachmann an sich aus der Literatur bekannten Methoden erfolgen. Insbesondere andere Veresterungskatalysatoren können verwendet werden.

Diese genannten Synthesen sind in WO 2010/149262, WO 2011/082770 und WO 2012/084118 beschrieben. Die Offenbarungen in den zitierten Literaturstellen gehören hiermit ausdrücklich auch zum Offenbarungsgehalt der vorliegenden Anmeldung.

Verbindungen der Formel (III) mit Rf = $CF_3$-$CF_2$-$CF_2$-O-CF($CF_3$)-CO-können bevorzugt durch Umsetzung von Diaminoolefinen mit den entsprechenden fluorierten Estern, Säuren oder Säurechloriden und Addition an die Doppelbindung zur Einführung der Gruppierung X nach dem Fachmann bekannten Methoden hergestellt werden.

Verbindungen der Formel (III) mit Rf = Rf' = $CF_3$-$CF_2$-$CF_2$-O-CF($CF_3$)-$CH_2$-O-$CH_2$-CHR'- können bevorzugt durch Umsetzung von Diaminoolefinen mit den entsprechenden fluorierten Estern, Säuren oder Säurechloriden, Addition an die Doppelbindung zur Einführung der Gruppierung X und Reduktion nach dem Fachmann bekannten Methoden hergestellt werden.

Diaminoolefine sind z.B. aus den entsprechenden Halogeniden über die Gabriel-Synthese zugänglich.

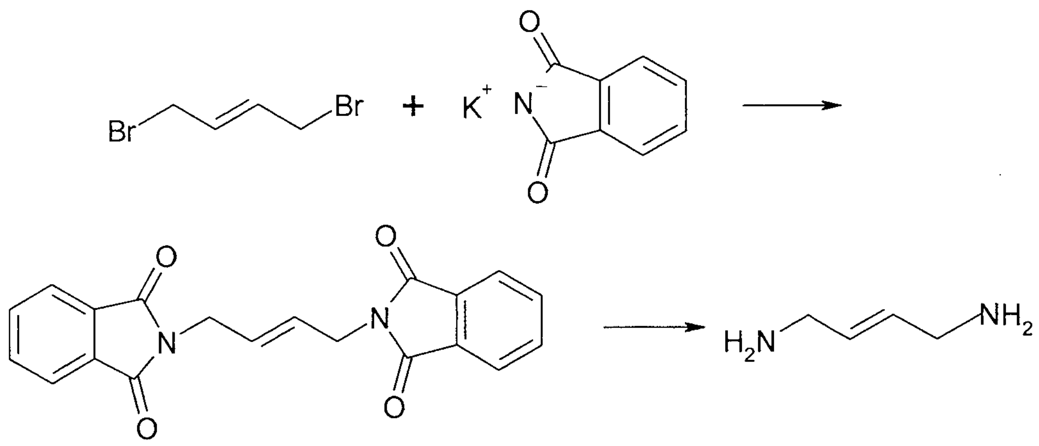

Mittels Metathese können symmetrische oder unsymmetrische Dicarbonsäureesterolefine hergestellt werden, die durch Aminolyse und anschließende Reduktion in geeignete Edukte überführt werden können.

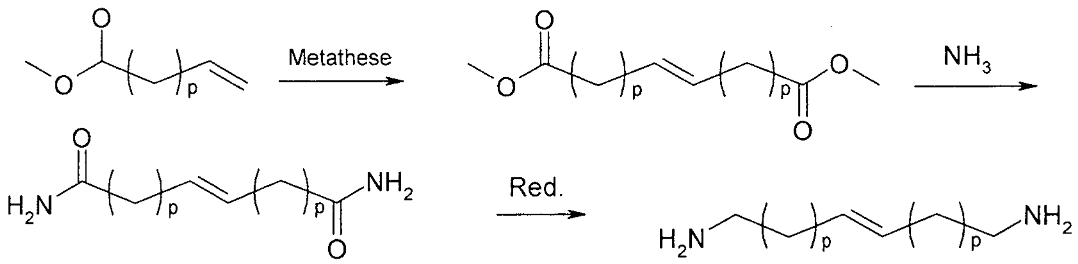


Verbindungen der Formel III-5 sind z.B. nach folgendem Syntheseschema darstellbar:

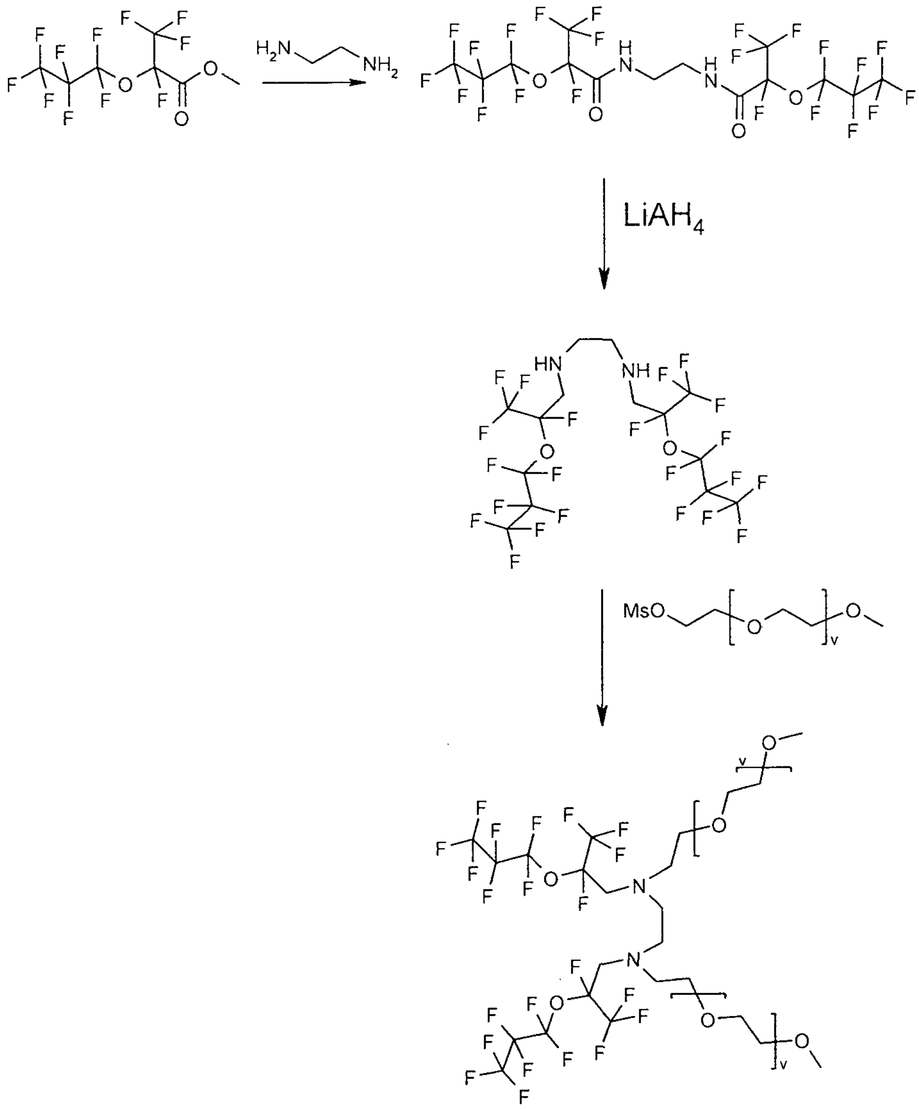


Die Herstellung weiterer erfindungsgemäßer Verbindungen kann analog zu den oben gezeigten beispielhaften Reaktionen erfolgen oder nach anderen dem Fachmann an sich aus der Literatur bekannten Methoden erfolgen.

Vorteile der erfindungsgemäßen Verbindungen können insbesondere sein:

- eine Oberflächenaktivität, die der konventioneller Kohlenwasserstoff-Tenside hinsichtlich Effizienz und/oder Effektivität gleich oder überlegen ist,
- biologische und/oder abiotische Abbaubarkeit der Substanzen ohne Bildung persistenter perfluorierter Abbauprodukte wie PFOA (Perfluoroctansäure) oder PFOS (Perfluoroctansulfonat),
- schwache Schaumwirkung und/oder geringe Schaumstabilisierung,
- gute Verarbeitbarkeit in Formulierungen und/oder
- Lagerstabilität.

Bevorzugt weisen die erfindungsgemäßen Verbindungen eine besondere Oberflächenaktivität auf. Die erfindungsgemäßen Verbindungen, insbesondere die Verbindungen der Formel (II), können gegenüber den Fluortensiden des Standes der Technik deutlich verbesserte Umwelteigenschaften aufweisen, da sie weder chemisch noch biologisch zu langkettigen PFCAs oder PFASs abbauen.

Ein zweiter Gegenstand der vorliegenden Erfindung ist die Verwendung der erfindungsgemäßen Verbindungen und den vorstehend beschriebenen bevorzugten Ausführungsformen als oberflächenaktive Mittel, beispielsweise zur Verbesserung des Verlaufsverhaltens und des Benetzungsvermögens von Coatingformulierungen. Fluortenside der Formeln (II) und (III) werden bevorzugt verwendet, insbesondere die genannten besonders bevorzugten Verbindungen.

Neben den erfindungsgemäßen Verbindungen können die erfindungsgemäßen Mischungen auch Lösemittel,

Additive, Hilfs- und Füllstoffe sowie nicht fluorierte Tenside enthalten. Beispielhaft seien genannt Silikonpartikel, Weichmacher und oberflächenmodifizierte Pigmente

Bevorzugte Einsatzgebiete sind beispielsweise die Verwendung der erfindungsgemäßen Fluortenside als Additive in Zubereitungen zur Oberflächenbeschichtung, wie Farben, Lacken, Schutzanstrichen, Spezialcoatings in elektronischen oder Halbleiter-Anwendungen (z.B. Photolacken, Top Antireflective Coatings, Bottom Antireflective Coatings) oder in optischen Anwendungen (z.B. photographischen Beschichtungen, Beschichtungen optischer Elemente), in Agrochemikalien, in Poliermitteln und Wachsen, z.B. für Mobiliar, Fußböden und Automobile, insbesondere in Bodenpolituren, in Feuerlöschmitteln, Schmierstoffen, in photolithographischen Verfahren, insbesondere in Immersionsphotolithographie-Verfahren, z.B. in Entwicklerlösungen, Spüllösungen, Immersionsölen und/oder in den Photoresists selbst, vor allem zur Herstellung von gedruckten Schaltungen oder in Additivzubereitungen zur Additivierung entsprechender Zubereitungen.

Darüber hinaus eignen sich die erfindungsgemäß als Tensid verwendbaren Verbindungen für Wasch- und Reinigungsanwendungen, sowie für eine Verwendung als Additive/Tenside in kosmetischen Produkten wie z. B. Haar- und Körperpflegeprodukten (z.B. Shampoos, Haarspülungen und Haarkonditionierern), Schaumbädern, Cremes oder Lotionen mit einer oder mehreren der folgenden Funktionen: Emulgatoren, Netzmittel, Schaummittel, Gleitmittel, Antistatikum, Erhöher der Resistenz gegen Hautfette.

Dabei werden die erfindungsgemäßen Fluortenside für die Anwendung üblicherweise in entsprechend ausgelegten Zubereitungen eingebracht. Übliche Einsatzkonzentrationen sind 0.01 - 1.0 Gew.-% der erfindungsgemäßen Tenside bezogen auf die Gesamtzubereitung. Entsprechende Mittel, enthaltend die erfindungsgemäßen Fluortensiden, sind ebenfalls Gegenstand der vorliegenden Erfindung. Bevorzugt enthalten solche Mittel einen für den jeweiligen Verwendungszweck geeigneten Träger, sowie gegebenenfalls weitere Aktivstoffe und/oder gegebenenfalls Hilfsstoffe. Bevorzugte Mittel sind Farb- und Lackzubereitungen, Feuerlöschmittel, Schmierstoffe, Wasch- und Reinigungsmittel und Enteiser oder Entwicklerlösungen, Spüllösungen, Immersionsöle und Photoresists für photolithograqphische Verfahren, insbesondere für Immersionsphotolithographie-Verfahren und insbesondere zur Herstellung gedruckter Schaltungen, Agrochemikalien, Bodenpolituren, kosmetische Produkte, kosmetische Produkte oder Hydrophobiermittel zur Textilausrüstung oder Glasbehandlung. Bei bevorzugten Mitteln handelt es sich dabei um Farb- und Lackzubereitungen und Druckfarben.

Außerdem sind auch wasserbasierte Lackformulierungen, die die erfindungsgemäßen Fluortenside allein oder im Gemische mit Additiven enthalten, Gegenstand der vorliegenden Erfindung. Bevorzugt werden Läckformulierungen auf Basis der folgenden synthetischen Filmbildner verwendet: Polykondensationsharze wie Alkydharze, gesättigt/ungesättigte Polyester, Polyamide/imide, Silikonharze; Phenolharze; Harnstoffharze und Melaminharze, Polyadditionsharze wie Polyurethane und Epoxidharze, Polymerisationsharze wie Polyolefine, Polyvinylverbindungen und Polyacrylate.

Außerdem sind die erfindungsgemäßen Fluortenside auch zum Einsatz in Lacken auf Basis von Naturstoffen und modifizierten Naturstoffen geeignet. Bevorzugt sind Lacke auf Basis von Ölen, Polysacchariden wie Stärke und Cellulose als auch auf Basis von Naturharzen wie cyclischen Oligoterpenen, Polyterpenen und/oder Schellack.

Die erfindungsgemäßen Fluortenside können sowohl in physikalisch härtenden (Thermoplaste) als auch in vernetzenden (Elastomere und Duromere) wässrigen Lacksystemen verwendet werden. Bevorzugt verbessern die erfindungsgemäßen Fluortenside die Verlaufs- und Benetzungseigenschaften der Lacksysteme.

Alle hier genannten Verwendungen erfindungsgemäß einzusetzender Fluortenside sind Gegenstand der vorliegenden Erfindung. Die jeweilige Anwendung von Fluortenside zu den genannten Zwecken ist dem Fachmann bekannt, so dass der Einsatz der erfindungsgemäß einzusetzenden Fluortenside keine Probleme bereitet.

Die vollständigen Offenbarungen aller aufgeführten Anmeldungen und Veröffentlichungen sind durch Bezugnahme in diese Anmeldung eingeführt. Für die vorliegende Erfindung bedeutet sowohl die Pluralform eines Begriffs als auch die Singularform eines Begriffs auch die jeweils andere Form, soweit nicht ausdrücklich anderes angegeben ist. Alle Merkmale der vorliegenden Erfindung können in jeder Art miteinander kombiniert werden, es sei denn dass sich bestimmte Merkmale ausschließen. Dies gilt vor allem für bevorzugte und besonders bevorzugte Merkmale. Weitere Merkmale, Vorteile und Varianten der Erfindung ergeben sich auch aus den Ansprüchen und Beispielen. Die folgenden Beispiele erläutern die vorliegende Erfindung näher, ohne den Schutzbereich zu beschränken.

## Beispiele

### Abkürzungen

| | |
|---|---|
| $CaCl_2$ | Calciumchlorid |
| $Et_2O$ | Diethylether |
| $LiAlH_4$ | Lithiumaluminiumhydrid |

| | |
|---|---|
| EtOAc | Essigester, Essigsäureethylester oder Ethansäureethylester |
| $Na_2SO_4$ | Natriumsulfat |
| $H_2O$ | Wasser |
| MTBE | tert-Butylmethylether |
| NaCl | Natriumchlorid |

**Beispiel 1: Synthese des 1H,1H-Perfluor(2-methyl-3-oxahexan-1-ol) (CAS 2101-3-71)**

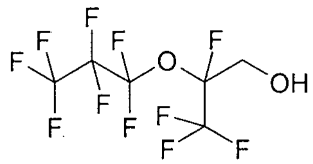

In einen trockenen 500 ml Vierhalskolben mit Metallkühler, $CaCl_2$ Trockenrohr, Tropftrichter und Thermometer werden 30 ml absol. $Et_2O$ vorlegen und über ein Septum 70 ml 1M $LiAlH_4$ Lösung (0,07 mol) in $Et_2O$ eintragen. In den Tropftrichter werden 44,30 g (0,12 mol) des Methyl Perfluor(2-methyl-3-oxahexanoats) (ABCR, Karlsruhe Germany) in 50 ml absol. $Et_2O$ vorgelegt.

Unter Rühren wird der Ester so zugetropft, so dass die Exothermie der Reaktion den Diethylether am Sieden hält. Nach vollendeter Zugabe rührt man das Reaktionsgemisch weitere 1,5 Stunden unter Rückfluss. Im Reaktionsverlauf entsteht eine trübe Dispersion. Der Ansatz wird im Eisbad abgekühlt. Die Zersetzung des überschüssigen $LiAlH_4$ erfolgt durch Zugabe von 10 ml EtOAc unter leichter Wärmetönung. Anschließend werden 10g $H_2O$ zugegeben, worauf ein flockiger Aluminiumhydroxid Niederschlag entsteht. Zu der Suspension wird innerhalb von 30 Minuten 78g 25%ige Schwefelsäure zugetropft, wobei eine klare zweiphasige Mischung entsteht. Die organische Phase wird abgetrennt und die wässrige Phase mit 3x 40 ml $Et_2O$ gewaschen. Die organischen Phasen werden vereinigt, mit 3x 40 ml $H_2O$ gewaschen und über $Na_2SO_4$ getrocknet. Der Ether wird abdestilliert und der Rückstand fraktioniert destilliert. Produkt: 31,8 g (Sdp. 57°C /100 mbar); Reinheit 91% (GC-MS); Ausbeute 73% der Theorie

**Beispiel 2: Synthese des 2-(2,3,3,3-Tetrafluoro-2-heptafluoropropyloxy-propoxy)-ethanol**

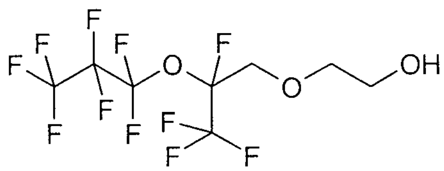

8,34 g (20 mmol) des 1H, 1H-Perfluor(2-methyl-3-oxahexan-1-ol)s werden zusammen mit 2,33 g (30 mmol) Ethylencarbonat und 0,33 g (2,4 mmol) Kaliumcarbonat 48 Stunden unter Rückfluss gerührt. Das Reaktionsgemisch wird mit je 25 ml halbkonzentrierter Salzsäure und MTBE versetzt und die Phasen getrennt.

Die wässrige Phase wird mit 2x25mL MTBE extrahiert und die vereinigten org. Phasen werden mit 1x30mL Wasser und 1x30mL gesättigter NaCl-Lösung gewaschen und über Natriumsulfat getrocknet. Das Lösemittel wird im Vakuum bei 400mbar und 40°C entfernt und der Rückstand fraktioniert destilliert.

Produkt: 6,5 g (Sdp. 37°C/ 0,5 mbar); Reinheit 85,3% (GC-MS); Ausbeute 64% der Theorie

Beispiel 3: **Synthese der Maleinsäureester mit kettenverlängerten Fluoralkoholen**

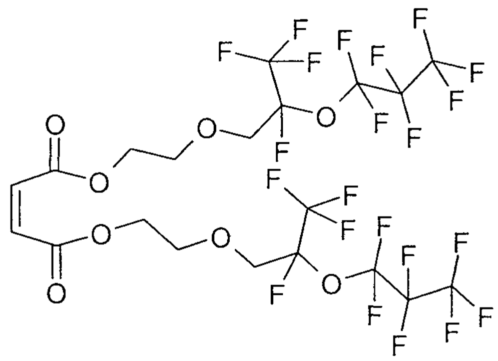

21 mmol kettenverlängerter Fluoralkohol, 10 mmol Maleinsäureanhydrid und 2 mmol p-Toluolsulfonsäure in 0,47 mol Toluol werden unter Rückfluss gerührt. Bei der Reaktion wird das freiwerdende Wasser mit Hilfe des Wasserabscheiders entfernt.
Die Reaktion wird mittels DC-Kontrolle verfolgt (Reaktionszeit 24-48 h). Es wird mit VE-Wasser versetzt und die Phasen getrennt.
Anschließend wird die wässrige Phase mit 3x100mL MTBE extrahiert, die vereinigten organischen Phasen mit 1x200mL Wasser und 1x200mL gesättigter NaCl-Lösung gewaschen und über Natriumsulfat getrocknet und filtriert. Das Lösemittel wird am Rotationsverdampfer abdestilliert. Das Rohprodukt wird durch eine fraktionierte Destillation aufgereinigt. Ausbeute für den (Z)-But-2-enedioic acid bis-[2-(2,3,3,3-tetrafluoro-2-heptafluoropropyloxy-propoxy)-ethyl] ester = 98%

**Beispiel 4: Synthese der Sulfosuccinate am Beispiel des Natrium-2-Sulfo-succinat bis-[2-(2,3,3,3-tetrafluoro-2-heptafluoropropyloxypropoxy)-ethyl] esters**

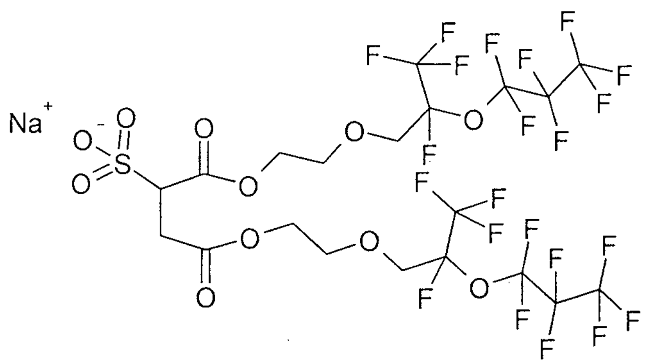

Zu einer Lösung des Maleinsäureesters (1 Moläquivalent) in 2-Propanol (100 Moläquivalente) wird bei 50°C Natriumhydrogensulfitlösung (5 Moläquivalente) getropft und anschließend noch für 72 Stunden am Rückfluss gerührt. Es wird mit 70mL Wasser versetzt und mit 3x50mL MTBE extrahiert. Die vereinigten organischen Phasen werden mit 1x50mL Wasser und gesättigter NaCl-Lösung gewaschen und über Natriumsulfat getrocknet. Das Lösemittel wird am Rotationsverdampfer entfernt. Ausbeute: 94 %

Beispiel 5: **Bestimmung der statischen Oberflächenspannung**

Es werden die statischen Oberflächenspannungen $\gamma$ von wässrigen Tensidlösungen mit verschiedenen Konzentrationen c (Gramm pro Liter) bestimmt.
Gerät: Tensiometer der Firma Dataphysics (Modell DCAT 11)
Temperatur der Messlösungen: 20°±0,2°C
Eingesetzte Messmethode: Messung der Oberflächenspannung mit der Wilhelmy Plattenmethode nach DIN EN 14370.
Platte: Platin, Länge= 19,9mm
Bei der Plattenmethode wird die Oberflächen- bzw. Grenzflächenspannung der Tensidlösung aus der auf die benetzte Länge einer Platte wirkenden Kraft nach folgender Formel berechnet.

$$\gamma = \frac{F}{L \cdot \cos\theta} = \frac{F}{L}$$

$\gamma$= Grenz- oder Oberflächenspannung; F= auf die Waage wirkende Kraft; L= benetzte Länge (19,9 mm); $\theta$= Kontaktwinkel)

Die Platte besteht aus angerautem Platin und wird also optimal benetzt, so dass der Kontaktwinkel $\theta$ nahe bei 0° liegt. Der Term cos $\theta$ erreicht daher annähernd den Wert 1, so dass nur noch die gemessene Kraft und die Länge der Platte berücksichtigt werden müssen.

Die Messwerte für das Sulfosuccinat gemäß Beispiel 4 sind in der Tabelle 1 wiedergegeben. Die Abbildung 1 zeigt die statische Oberflächenspannung in Abhängigkeit von der Konzentration für das Sulfosuccinat gemäß Beispiel 4.

Tabelle 1:

| **c [g/L]** | **$\gamma$[mN/m]** |
|---|---|
| 0,0003 | 57,8 |
| 0,0005 | 50,9 |
| 0,0008 | 44,8 |
| 0,00132 | 39,2 |
| 0,00216 | 35,2 |
| 0,00354 | 31,8 |
| 0,00579 | 28,6 |
| 0,00949 | 24,3 |
| 0,01554 | 20,6 |
| 0,02545 | 17,7 |
| 0,04169 | 16,8 |
| 0,06828 | 17,2 |
| 0,11183 | 18,8 |
| 0,18316 | 18,5 |
| 0,3 | 17,4 |
| 0,5 | 16,4 |
| 1 | 16,1 |

**Beispiel 6: Bestimmung der dynamischen Oberflächenspannung**

Es wird die dynamische Oberflächenspannung $\gamma$ einer 0.1%igen (Gewichtsprozent) wässrigen Lösung der zu untersuchenden Verbindung bestimmt.

Eingesetzte Messmethode: Messung der Oberflächenspannung mit der Blasend ruckmethode

Gerät: Tensiometer der Firma SITA (Modell t 60)

Temperatur der Messlösungen: 20°C ± 0,2°C

Bei der Messung der dynamischen Oberflächenspannung werden Luftblasen mit verschiedenen Geschwindigkeiten durch eine Kapillare in die Tensidlösung gedrückt. Aus der dabei auftretenden Druckänderung kann die Oberflächenspannung in Abhängigkeit von der Blasenlebensdauer mit folgender Gleichung bestimmt werden:

$$\gamma = \frac{r(p_{max} - \rho \cdot g \cdot h)}{2}$$

$p_{max}$= Maximaldruck, $\rho$= Dichte der Flüssigkeit, h= Eintauchtiefe, r= Radius der Kapillare

Die Messwerte für das Sulfosuccinat gemäß Beispiel 4 sind in der Tabelle 2 wiedergegeben. Die Abbildung 2 zeigt die

dynamische Oberflächenspannung in Abhängigkeit von der Blasenlebensdauer für das Sulfosuccinat gemäß Beispiel 4.

Tabelle 2

| Blasenlebensdauer [ms] | $\gamma$ [mN/m] |
|---|---|
| 32 | 70,6 |
| 38 | 70,8 |
| 52 | 70,5 |
| 65 | 69,8 |
| 84 | 69,3 |
| 111 | 68,6 |
| 144 | 67,4 |
| 189 | 66 |
| 243 | 64 |
| 311 | 61,2 |
| 407 | 56,7 |
| 529 | 51,2 |
| 678 | 44,3 |
| 900 | 35 |
| 1033 | 29 |
| 1318 | 23,1 |
| 1798 | 19,4 |
| 2615 | 18,4 |
| 9738 | 15,9 |
| 12349 | 15,1 |
| 16176 | 14,9 |
| 19920 | 14,7 |
| 31841 | 14,4 |
| 33091 | 14,3 |
| 47713 | 14,2 |
| 60227 | 14,1 |

## Patentansprüche

**1.** Verbindungen der Formel (I), (II) oder (III)

$$(R_f\text{-}A_a\text{-}C_c)_n\text{-}(\text{Spacer})_m\text{-}X_o \qquad (I)$$

wobei

für Rf = $CF_3$-$CF_2$-$CF_2$-O-$CF(CF_3)$-$CH_2$,
A = -O-$CH_2$-CHR'-,
R' = H oder C1-C4 Alkyl,
C = C1-C4 Alkylen, O oder -OCO-,
Spacer = eine lineare oder verzweigte, gegebenenfalls mit Heteroatomen versehene, Kohlenwasserstoff-Einheit,

X eine anionische, kationische, nicht-ionische oder amphotere Gruppe ist,
a = 1,
n = 2 oder 3,
c = 1,
m = 1, und
o = 1 oder 2,
und
für Rf = $CF_3$-$CF_2$-$CF_2$-O-CF($CF_3$)-CO-,
C = -NR"- mit R" = H, Alkyl, oder -($CH_2$CHR"')$_{m'}$-R"" mit m' = eine ganze Zahl aus dem Bereich von 1 bis 100 und R"' = H oder C1-C4-Alkyl und R"" = Alkyl, wobei ein oder mehrere nicht benachbarte C-Atome durch O ersetzt sein können
Spacer = lineares oder verzweigtes Alkylen,
X eine anionische, kationische, nicht-ionische oder amphotere Gruppe ist,
a = 0,
n = 2 oder 3,
c = 1,
m =1,und
o = 1 oder 2, und o = 0 wenn R" = -($CH_2$CHR"')$_{m'}$-R"";

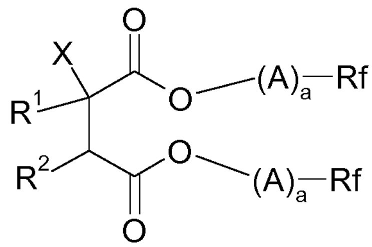

(II)

wobei

Rf = $CF_3$-$CF_2$-$CF_2$-O-CF($CF_3$)-$CH_2$--,
A = -O-$CH_2$-CHR'-,
R' = H, $CH_3$, $C_2H_5$
a = 1 ist
X eine anionische, kationische, nicht-ionische oder amphotere Gruppe ist,
$R^1$ = Wasserstoff oder -$CH_2$-COO-(A)$_a$-Rf,
$R^2$ = Wasserstoff, -$CH_2$-COO-(A)$_a$-Rf oder X, ist;

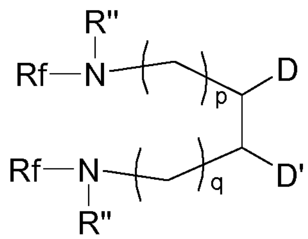

(III)

wobei

Rf = $CF_3$-$CF_2$-$CF_2$-O-CF($CF_3$)-CO- oder = Rf' mit
Rf' = $CF_3$-$CF_2$-$CF_2$-O-CF($CF_3$)-$CH_2$-O-$CH_2$-CHR'-,
p = 1-10,
q = 1-10 und
R' und R" die für Formel (I) beschriebenen Bedeutungen haben,
D und/oder D' = H oder X, wobei X eine anionische, kationische, nicht-ionische oder amphotere Gruppe ist und mindestens eine Gruppe gleich X ist, wenn R" = H oder Alkyl.

2. Verbindungen gemäß Anspruch 1, **dadurch gekennzeichnet, dass** sie der Formel (II-a) entsprechen:

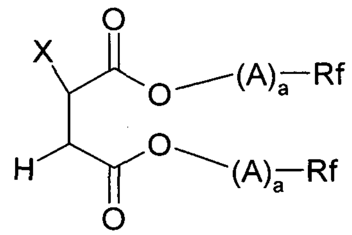

(II-a)

wobei

Rf = $CF_3$-$CF_2$-$CF_2$-O-CF($CF_3$)-$CH_2$--,
A = -O-$CH_2$-CHR'-,
R' = H, $CH_3$, $C_2H_5$
a = 1 ist
X eine anionische, kationische, nicht-ionische oder amphotere Gruppe ist.

3. Verbindungen gemäß Anspruch 1, **dadurch gekennzeichnet, dass** sie der Formel (II-b) entsprechen:

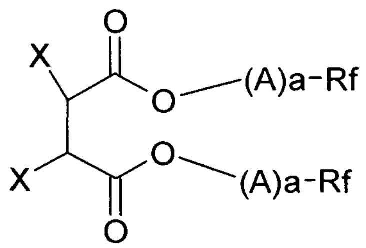

(II-b)

wobei

Rf = $CF_3$-$CF_2$-$CF_2$-O-CF($CF_3$)-$CH_2$--,
A = -O-$CH_2$-CHR'-,
R' = H, $CH_3$, $C_2H_5$
a = 1 ist
X eine anionische, kationische, nicht-ionische oder amphotere Gruppe ist.

4. Verbindungen gemäß Anspruch 1, **dadurch gekennzeichnet, dass** sie der Formel (II-c) entsprechen:

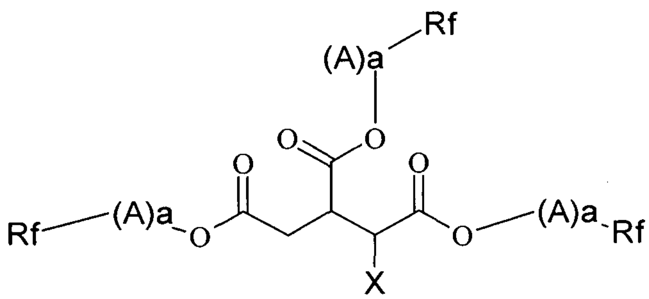

(II-c)

wobei

Rf = $CF_3$-$CF_2$-$CF_2$-O-CF($CF_3$)-$CH_2$--,
A = -O-$CH_2$-CHR'-,
R' = H, $CH_3$, $C_2H_5$
a = 1 ist

X eine anionische, kationische, nicht-ionische oder amphotere Gruppe ist.

5. Verbindungen Anspruch 1, **dadurch gekennzeichnet, dass** sie der Formel (II-d) entsprechen:

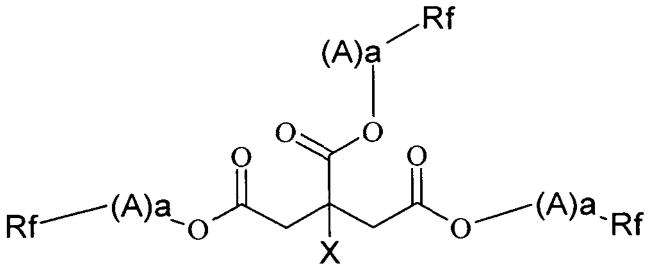

(II-d)

wobei

Rf = $CF_3$-$CF_2$-$CF_2$-O-CF($CF_3$)-$CH_2$--,
A = -O-$CH_2$-CHR'-,
R' = H, $CH_3$, $C_2H_5$
a = 1 ist
X eine anionische, kationische, nicht-ionische oder amphotere Gruppe ist.

6. Verbindungen gemäß Anspruch 1, **dadurch gekennzeichnet, dass** in Formel (III) Rf = $CF_3$-$CF_2$-$CF_2$-O-CF($CF_3$)-CO-,R" = H oder Alkyl und D und/oder D' = X ist.

7. Verbindungen gemäß einem oder mehreren der Ansprüche 1 bis 6, **dadurch gekennzeichnet, dass** X gleich -$SO_3^-$, -$OSO_3^-$, -$PO_3^{2-}$, -OPO3$^{2-}$, -CH(OH)-$CH_2$-NH-Sach mit Sach = verschiedene Zucker oder -Y-($CH_2$-$CH_2$-O)$_v$-R mit Y = S, O oder NH und R = H oder $CH_3$ und v = 1-15 ist.

8. Verbindungen gemäß einem oder mehreren der Ansprüche 1 bis 6, **dadurch gekennzeichnet, dass** X gleich -CH(OH)-$CH_2$-NH-Sach mit Sach = verschiedene Zucker oder die Gruppe -Y-($CH_2$-$CH_2$-O)$_v$-R mit Y = S, O oder NH und R = H oder $CH_3$ und v = 1-15 ist.

9. Verbindungen gemäß einem oder mehreren der Ansprüche 1 bis 5, **dadurch gekennzeichnet, dass** sie einer der Formeln (II-a/1) bis (II-a/4) entsprechen

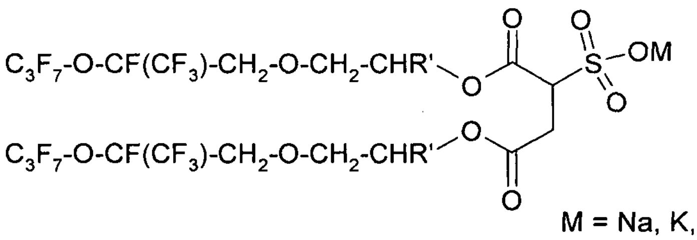

(II-a/1)

M = Na, K, $NH_4$

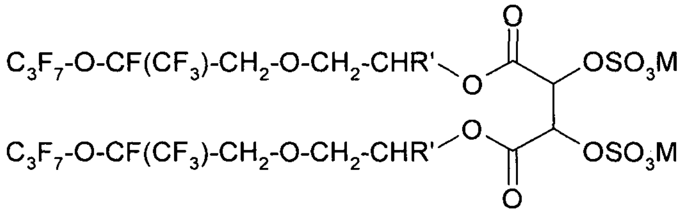

(II-a/2)

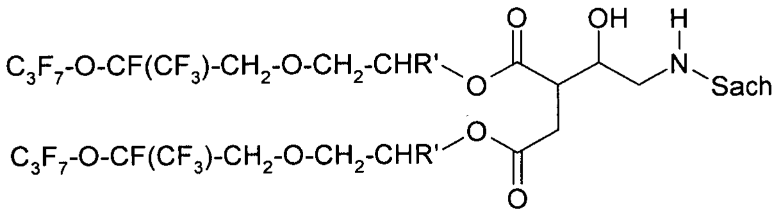

(II-a/3)

Sach = verschiedene Zucker

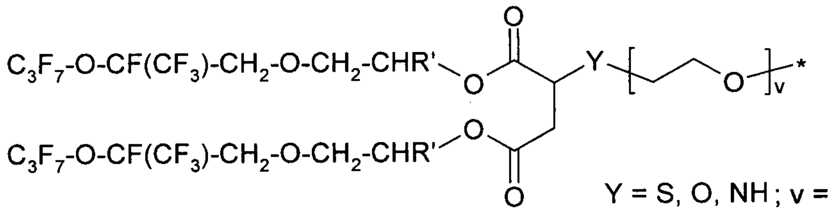

(II-a/4)

Y = S, O, NH ; v = 1-15

**10.** Verbindungen gemäß einem oder mehreren der Ansprüche 1 und 6, **dadurch gekennzeichnet, dass** sie einer der Formeln (III-1) bis (III-9) entsprechen

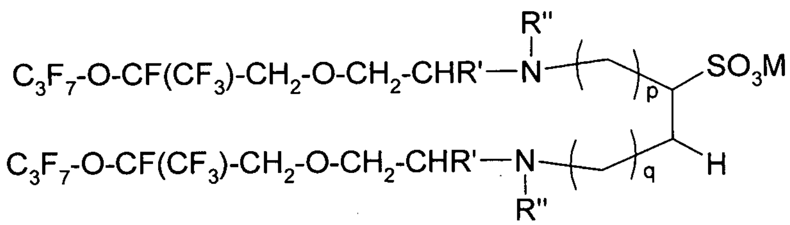

(III-1)

M = Na, K, NH4

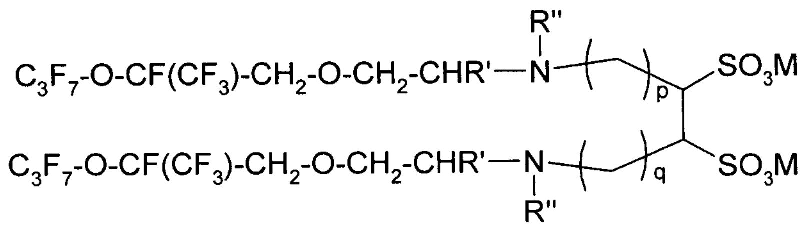

(III-2)

M = Na, K, NH4

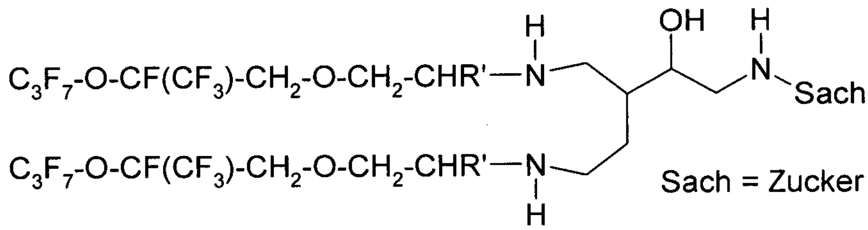

(III-3)

Sach = Zucker

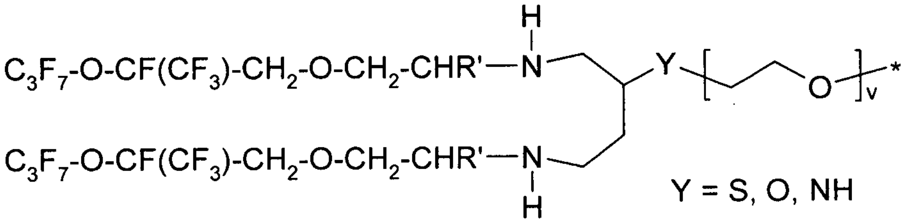

(III-4)

Y = S, O, NH

v = 1-100

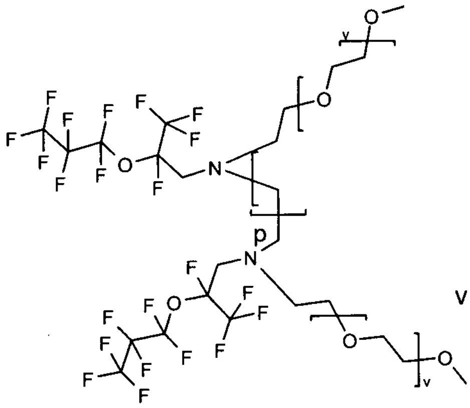

(III-5)

v = 1-100

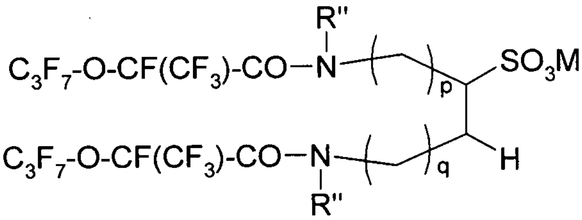

(III-6)

M = Na, K, NH4

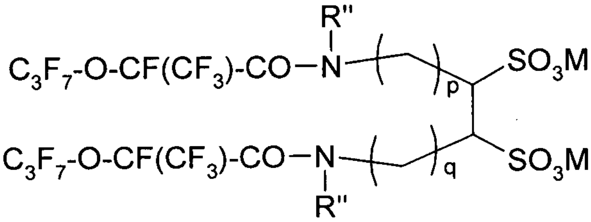

(III-7)

M = Na, K, NH4

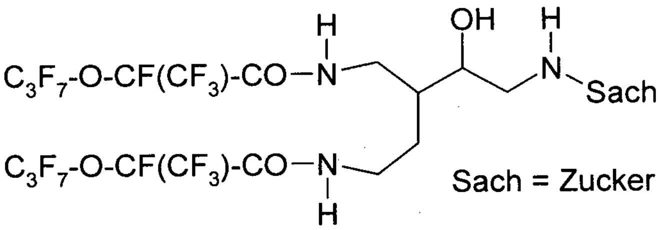

(III-8)

Sach = Zucker

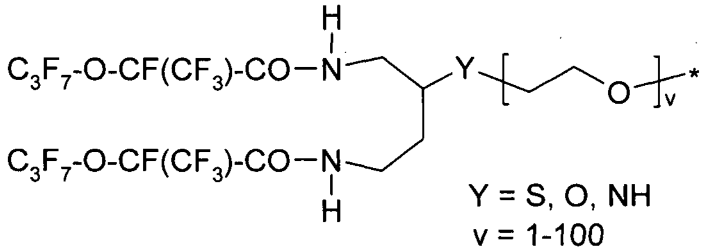

Y = S, O, NH
v = 1-100

(III-9)

**11.** Verwendung von Verbindungen gemäß einem oder mehreren der Ansprüche 1 bis 10 als Additive in Farben, Lacken, Druckfarben, Schutzanstrichen, Spezialcoatings in elektronischen oder optischen Anwendungen, Photolacken, Top Antireflective Coatings oder Bottom Antireflective Coatings, Entwicklerlösungen und Waschlösungen und Photoresists für photolithograqphische Verfahren, kosmetischen Produkten, Agrochemikalien, Bodenpolituren, photographischen Beschichtungen oder Beschichtungen optischer Elemente.

**12.** Mittel enthaltend Verbindungen gemäß einem oder mehreren der Ansprüche 1 bis 10 und einen für den jeweiligen Verwendungszweck geeigneten Träger sowie ggf. weitere spezifische Aktivstoffe.

**13.** Mittel nach Anspruch 12, **dadurch gekennzeichnet, dass** es sich bei dem Mittel um Farb- und Lackzubereitungen, Feuerlöschmittel, Schmierstoffe, Wasch- und Reinigungsmittel, Enteiser, Entwicklerlösungen und Waschlösungen und Photoresists für photolithograqphische Verfahren, kosmetische Produkte, Agrochemikalien, Bodenpolituren oder Hydrophobiermittel zur Textilausrüstung oder Glasbehandlung handelt.

## Claims

**1.** Compounds of the formula (I), (II) or (III)

$$(R_f\text{-}A_a\text{-}C_c)_n\text{-}(\text{spacer})_m\text{-}X_o \qquad (I)$$

where

for Rf = $CF_3$-$CF_2$-$CF_2$-O-CF($CF_3$)-$CH_2$-,
A = -O-$CH_2$-CHR'-,
R' = H or C1-C4 alkyl,
C = C1-C4 alkylene, O, or -OCO-,
spacer = a linear or branched hydrocarbon unit, optionally provided with heteroatoms,
X is an anionic, cationic, nonionic or amphoteric group,
a = 1,
n = 2 or 3,
c = 1,
m = 1, and
o = 1 or 2,
and
for Rf = $CF_3$-$CF_2$-$CF_2$-O-CF($CF_3$)-CO-,
C = -NR"-, where R" = H, alkyl or -($CH_2$CHR"')$_{m'}$-R"", where m' = an integer from the range from 1 to 100 and R"' = H or C1-C4-alkyl and R"" = alkyl, where one or more non-adjacent C atoms may be replaced by O,
spacer = linear or branched alkylene,
X is an anionic, cationic, nonionic or amphoteric group,
a = 0,
n = 2 or 3,
c = 1,
m = 1, and
o = 1 or 2, and o = 0 if R" = -($CH_2$CHR"')$_{m'}$-R"";

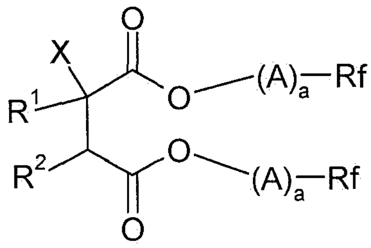

(II)

where

Rf = $CF_3$-$CF_2$-$CF_2$-O-CF($CF_3$)-$CH_2$-,
A = -O-$CH_2$-CHR'-,
R' = H, $CH_3$, $C_2H_5$,
a = 1,
X is an anionic, cationic, nonionic or amphoteric group,
$R^1$ = hydrogen or -$CH_2$-COO-$(A)_a$-Rf,
$R^2$ = hydrogen, -$CH_2$-COO-$(A)_a$-Rf or X,

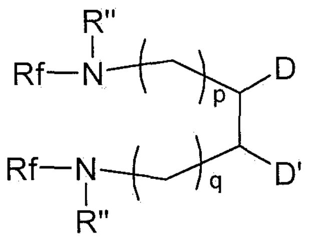

(III)

where

Rf = $CF_3$-$CF_2$-$CF_2$-O-CF($CF_3$)-CO- or = Rf', where
Rf' = $CF_3$-$CF_2$-$CF_2$-O-CF($CF_3$)-$CH_2$-O-$CH_2$-CHR'-,
p = 1-10,
q = 1-10 and
R' and R" have the meanings described for formula (I),
D and/or D' = H or X, where X is an anionic, cationic, nonionic or amphoteric group and at least one group is equal to X if R" = H or alkyl.

**2.** Compounds according to Claim 1, **characterised in that** they conform to the formula (II-a):

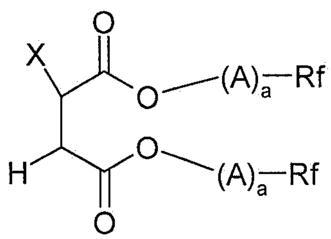

(II-a)

where

Rf = $CF_3$-$CF_2$-$CF_2$-O-CF($CF_3$)-$CH_2$-,
A = -O-$CH_2$-CHR'-,
R' = H, $CH_3$, $C_2H_5$,
a = 1,

X is an anionic, cationic, non-ionic or amphoteric group.

3. Compounds according to Claim 1, **characterised in that** they conform to the formula (II-b):

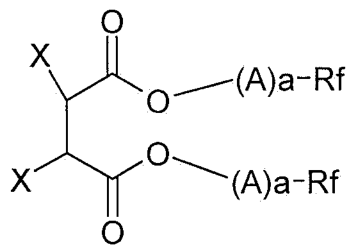

(II-b)

where

Rf = $CF_3$-$CF_2$-$CF_2$-O-$CF(CF_3)$-$CH_2$-,
A = -O-$CH_2$-CHR'-,
R' = H, $CH_3$, $C_2H_5$,
a = 1,
X is an anionic, cationic, nonionic or amphoteric group.

4. Compounds according to Claim 1, **characterised in that** they conform to the formula (II-c):

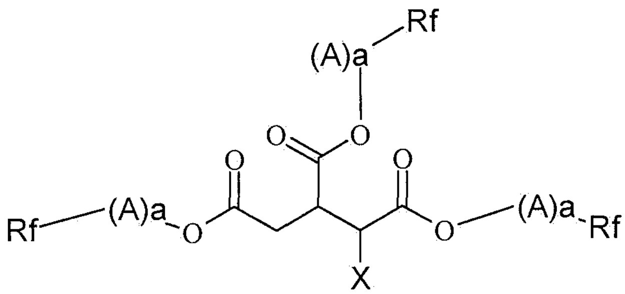

(II-c)

where

Rf = $CF_3$-$CF_2$-$CF_2$-O-$CF(CF_3)$-$CH_2$-,
A = -O-$CH_2$-CHR'-,
R' = H, $CH_3$, $C_2H_5$,
a = 1,
X is an anionic, cationic, nonionic or amphoteric group.

5. Compounds according to Claim 1, **characterised in that** they conform to the formula (II-d):

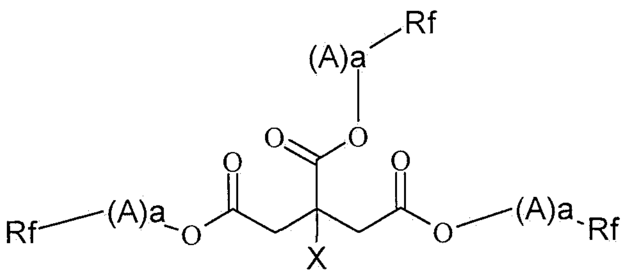

(II-d)

where

Rf = $CF_3$-$CF_2$-$CF_2$-O-$CF(CF_3)$-$CH_2$-,
A = -O-$CH_2$-CHR'-,

R' = H, $CH_3$, $C_2H_5$,
a = 1,
X is an anionic, cationic, nonionic or amphoteric group.

**6.** Compounds according to Claim 1, **characterised in that**, in formula (III), Rf = $CF_3$-$CF_2$-$CF_2$-O-CF($CF_3$)-CO-,R" = H or alkyl and D and/or D'=X.

**7.** Compounds according to one or more of Claims 1 to 6, **characterised in that** X is equal to -$SO_3^-$, -$OSO_3^-$, -$PO_3^{2-}$, -OPO3$^{2-}$, -CH(OH)-$CH_2$-NH-Sach, where Sach = various sugars or -Y-($CH_2$-$CH_2$-O)$_v$-R, where Y = S, O or NH and R = H or $CH_3$ and v = 1-15.

**8.** Compounds according to one or more of Claims 1 to 6, **characterised in that** X is equal to -CH(OH)-$CH_2$-NH-Sach, where Sach = various sugars or the group -Y-($CH_2$-$CH_2$-O)$_v$-R, where Y = S, O or NH and R = H or $CH_3$ and v = 1-15.

**9.** Compounds according to one or more of Claims 1 to 5, **characterised in that** they conform to one of the formulae (II-a/1) to (II-a/4)

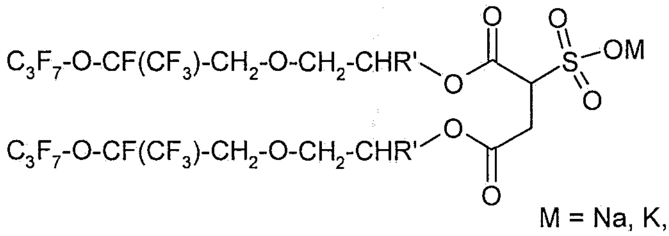

(II-a/1)

M = Na, K, $NH_4$

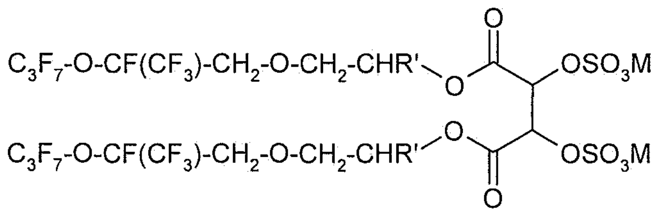

(II-a/2)

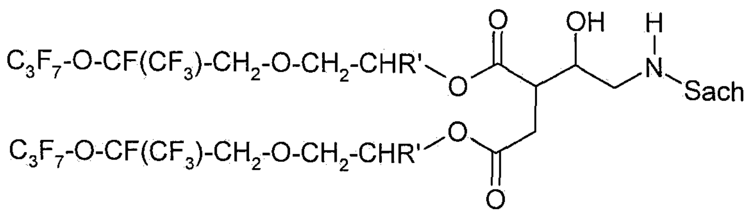

(II-a/3)

Sach = various sugars

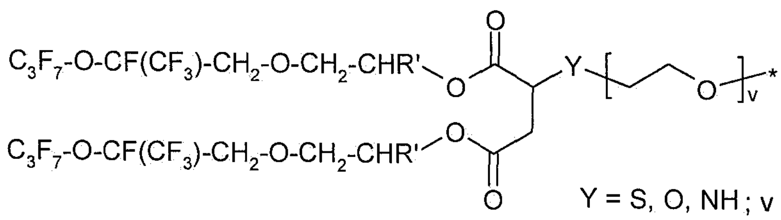

(II-a/4)

Y = S, O, NH ; v = 1-15

**10.** Compounds according to one or more of Claims 1 and 6, **characterised in that** they conform to one of the formulae (III-1) to (III-9)

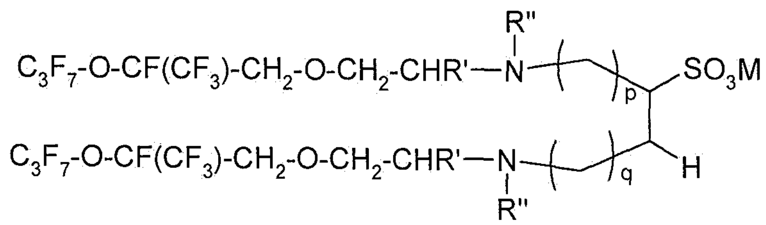

(III-1)

M = Na, K, NH4

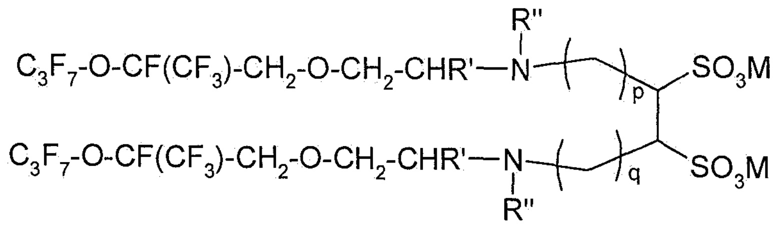

(III-2)

M = Na, K, NH4

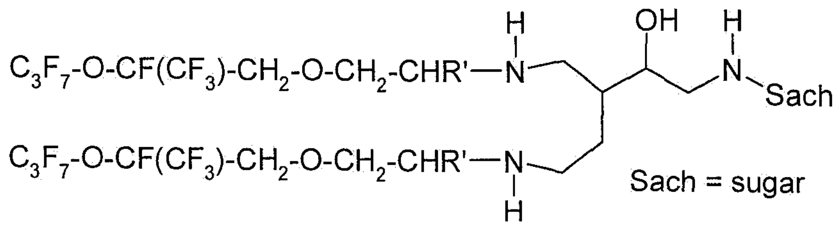

Sach = sugar

(III-3)

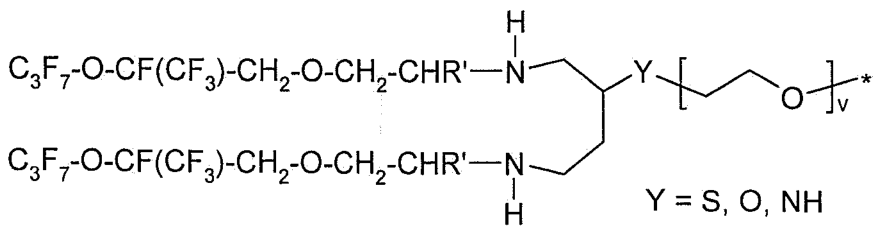

(III-4)

Y = S, O, NH

v = 1-100

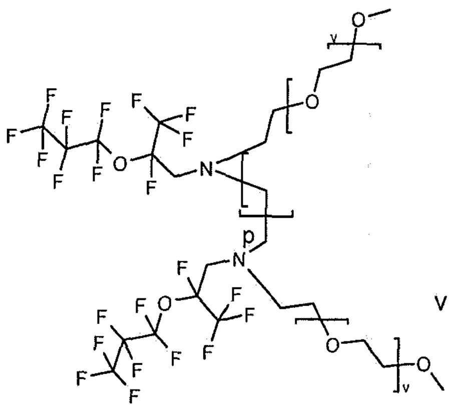

(III-5)

v = 1-100

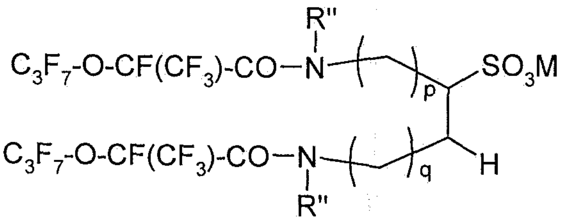

(III-6)

M = Na, K, NH4

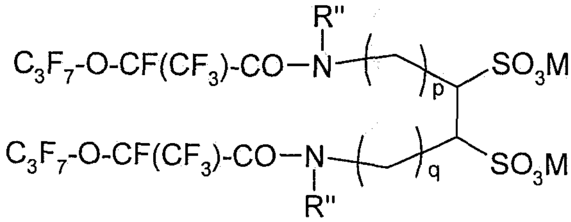

(III-7)

M = Na, K, NH4

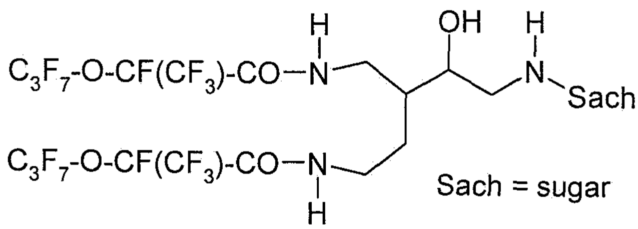

(III-8)

Sach = sugar

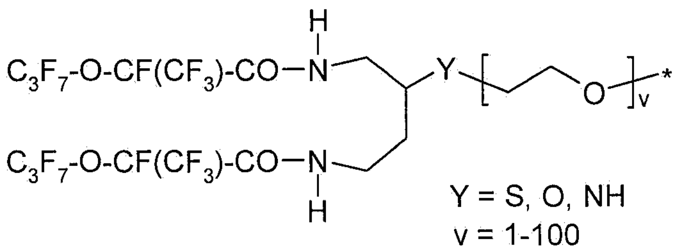

(III-9)

Y = S, O, NH
v = 1-100

**11.** Use of compounds according to one or more of Claims 1 to 10 as additives in paints, coatings, printing inks, protective coatings, special coatings in electronic or optical applications, photoresists, top antireflective coatings or bottom antireflective coatings, developer solutions and washing solutions and photoresists for photolithographic processes, cosmetic products, agrochemicals, floor polishes, photographic coatings or coatings of optical elements.

**12.** Composition comprising compounds according to one or more of Claims 1 to 10 and a vehicle which is suitable for the respective application, and optionally further specific active substances.

**13.** Composition according to Claim 12, **characterised in that** the composition is paint and coating preparations, fire-extinguishing compositions, lubricants, washing compositions and detergents, de-icers, developer solutions and washing solutions and photoresists for photolithographic processes, cosmetic products, agrochemicals, floor polishes or hydrophobicising compositions for textile finishing or glass treatment.

**Revendications**

**1.** Composés de formule (I), (II) ou (III)

$$(R_f\text{-}A_a\text{-}C_c)_n\text{-}(\text{espaceur})_m\text{-}X_o \qquad (I)$$

dans lesquels

pour Rf= $CF_3$-$CF_2$-$CF_2$-O-CF($CF_3$)-$CH_2$-,
A = -O-$CH_2$-CHR'-,
R' = H ou C1-C4 alkyle,
C = C1-C4 alkylène, O, ou -OCO-,
espaceur = un motif hydrocarboné linéaire ou ramifié, éventuellement pourvu d'hétéroatomes,
X est un groupement anionique, cationique, non ionique ou amphotère,
a = 1,
n = 2 ou 3,
c = 1,
m = 1, et
o = 1 ou 2,
et
pour Rf = $CF_3$-$CF_2$-$CF_2$-O-CF($CF_3$)-CO-,
C = -NR"-, où R" = H, alkyle ou -($CH_2$CHR"')$_{m'}$-R"", où m' = un nombre entier dans la plage allant de 1 à 100 et R"' = H ou C1-C4-alkyle et R"" = alkyle, où un ou plusieurs atomes de C non adjacents peuvent être remplacés par O,
espaceur = alkylène linéaire ou ramifié,
X est un groupement anionique, cationique, non ionique ou amphotère,
a = 0,
n = 2 ou 3,
c = 1,
m = 1, et
o = 1 ou 2, et o = 0 si R" = -($CH_2$CHR"')$_{m'}$-R"";

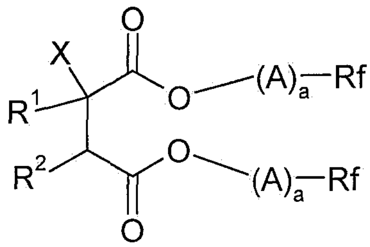

(II)

où

Rf = $CF_3$-$CF_2$-$CF_2$-O-CF($CF_3$)-$CH_2$-,
A = -O-$CH_2$-CHR'-,
R' = H, CH3, $C_2H_5$,
a = 1,
X est un groupement anionique, cationique, non ionique ou amphotère,
$R^1$ = hydrogène ou -$CH_2$-COO-(A)$_a$-Rf,
$R^2$ = hydrogène, -$CH_2$-COO-(A)$_a$-Rf ou X,

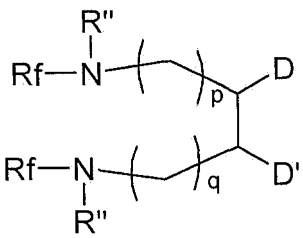

(III)

où

Rf = $CF_3$-$CF_2$-$CF_2$-O-CF($CF_3$)-CO- ou = Rf', où Rf' = $CF_3$-$CF_2$-$CF_2$-O-CF($CF_3$)-$CH_2$-O-$CH_2$-CHR'-,
p = 1-10,

q = 1-10 et
R' et R" revêtent les significations décrites pour la formule (I),
D et/ou D' = H ou X, où X est un groupement anionique, cationique, non ionique ou amphotère et au moins un groupement est égal à X si R" = H ou alkyle.

**2.** Composés selon la revendication 1, **caractérisés en ce qu'**ils répondent à la formule (II-a) :

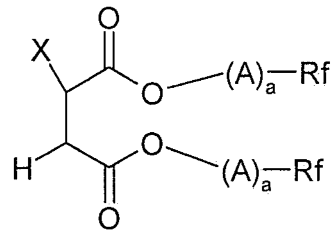

(II-a)

où

Rf = $CF_3$-$CF_2$-$CF_2$-O-CF($CF_3$)-$CH_2$-,
A = -O-$CH_2$-CHR'-,
R' = H, $CH_3$, $C_2H_5$,
a = 1,
X est un groupement anionique, cationique, non ionique ou amphotère.

**3.** Composés selon la revendication 1, **caractérisés en ce qu'**ils répondent à la formule (II-b) :

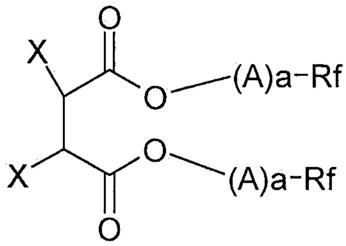

(II-b)

où

Rf = $CF_3$-$CF_2$-$CF_2$-O-CF($CF_3$)-$CH_2$-,
A = -O-$CH_2$-CHR'-,
R' = H, $CH_3$, $C_2H_5$,
a = 1,
X est un groupement anionique, cationique, non ionique ou amphotère.

**4.** Composés selon la revendication 1, **caractérisés en ce qu'**ils répondent à la formule (II-c) :

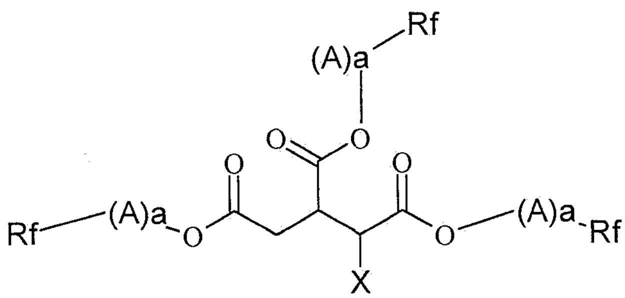

(II-c)

où

$Rf = CF_3-CF_2-CF_2-O-CF(CF_3)-CH_2-$,
$A = -O-CH_2-CHR'-$,
$R' = H, CH_3, C_2H_5$,
$a = 1$,
X est un groupement anionique, cationique, non ionique ou amphotère.

5. Composés selon la revendication 1, **caractérisés en ce qu'**ils répondent à la formule (II-d) :

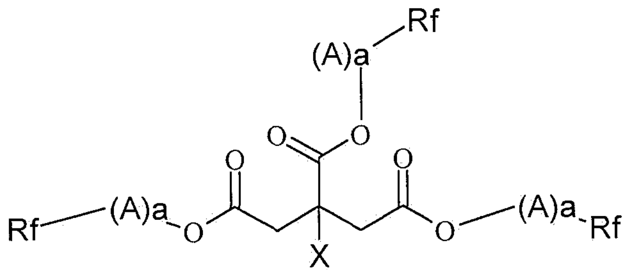

(II-d)

où

$Rf = CF_3-CF_2-CF_2-O-CF(CF_3)-CH_2-$,
$A = -O-CH_2-CHR'-$,
$R' = H, CH_3, C_2H_5$,
$a = 1$,
X est un groupement anionique, cationique, non ionique ou amphotère.

6. Composés selon la revendication 1, **caractérisés en ce que**, dans la formule (III), $Rf = CF_3-CF_2-CF_2-O-CF(CF_3)-CO-$, R" = H ou alkyle et D et/ou D'=X.

7. Composés selon l'une ou plusieurs parmi les revendications 1 à 6, **caractérisés en ce que** X est égal à $-SO_3^-$, $-OSO_3^-$, $-PO_3^{2-}$, $-OPO_3^{2-}$, $-CH(OH)-CH_2-NH-Sach$, où Sach = divers sucres ou $-Y-(CH_2-CH_2-O)_v-R$, où Y = S, O ou NH et R = H ou $CH_3$ et v = 1-15.

8. Composés selon l'une ou plusieurs parmi les revendications 1 à 6, **caractérisés en ce que** X est égal à $-CH(OH)-CH_2-NH-Sach$, où Sach = divers sucres ou le groupement $-Y-(CH_2-CH_2-O)_v-R$, où Y = S, O ou NH et R = H ou $CH_3$ et v = 1-15.

9. Composés selon l'une ou plusieurs parmi les revendications 1 à 5, **caractérisés en ce qu'**ils répondent à l'une des formules (II-a/1) à (II-a/4)

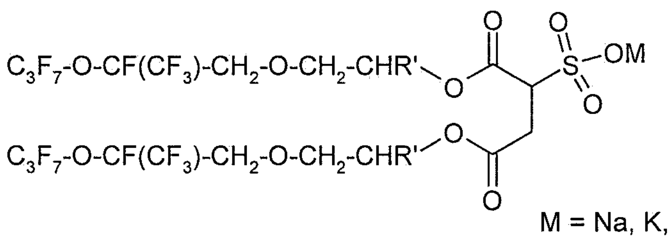

(II-a/1)

M = Na, K, $NH_4$

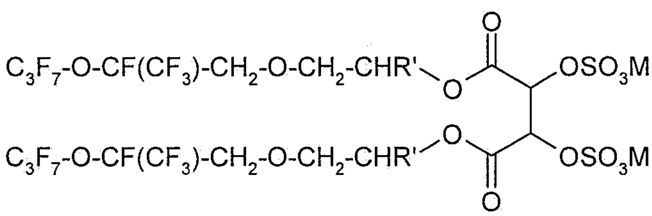

(II-a/2)

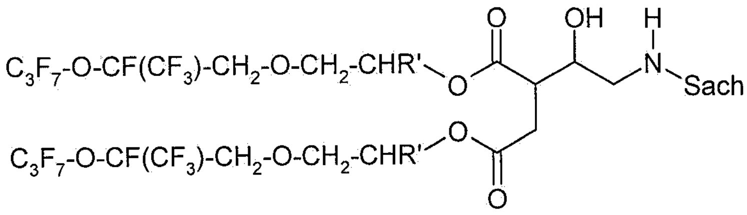

(II-a/3)

Sach = divers sucres

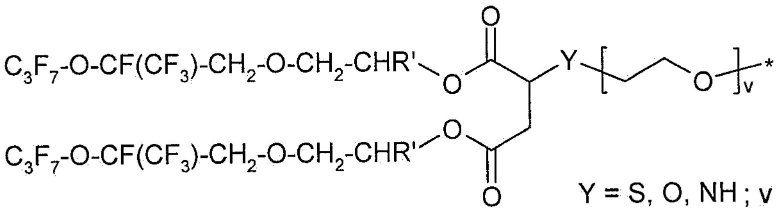

(II-a/4)

Y = S, O, NH ; v = 1-15

**10.** Composés selon l'une ou plusieurs parmi les revendications 1 et 6, **caractérisés en ce qu'**ils répondent à l'une des formules (III-1) à (III-9)

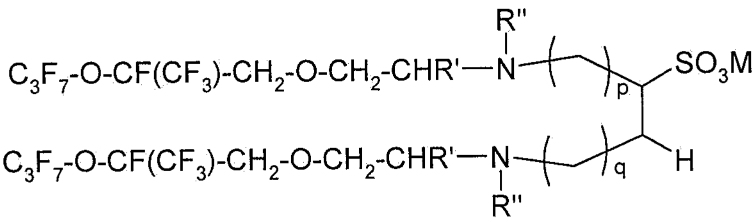

(III-1)

M = Na, K, NH4

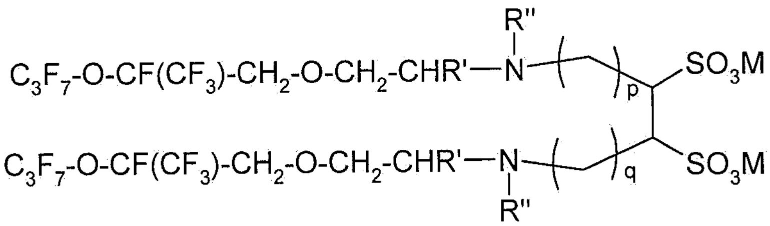

(III-2)

M = Na, K, NH4

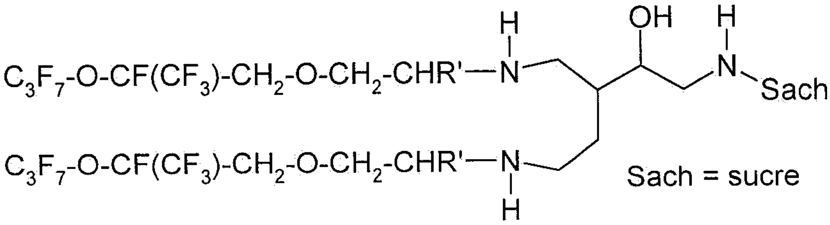

(III-3)

Sach = sucre

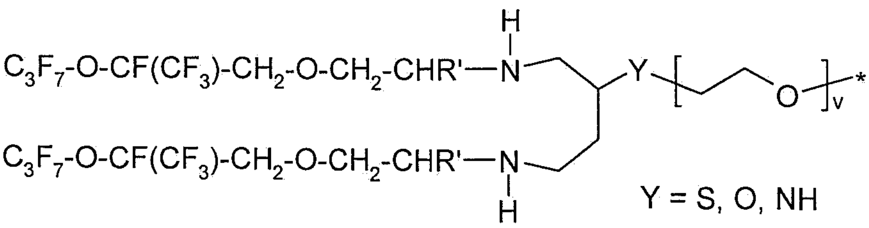

(III-4)

Y = S, O, NH

v = 1-100

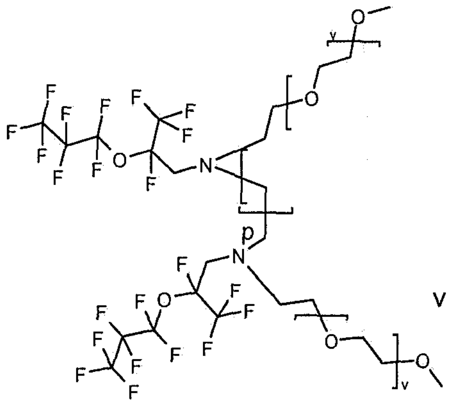

(III-5)

v = 1-100

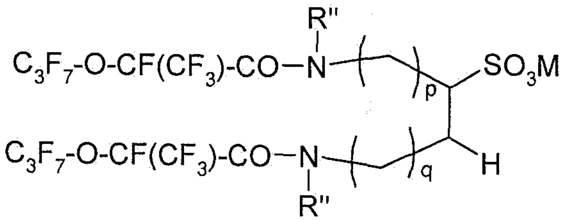

(III-6)

M = Na, K, NH4

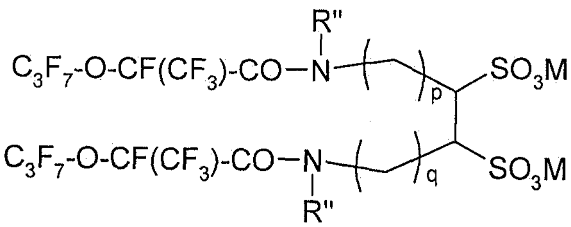

(III-7)

M = Na, K, NH4

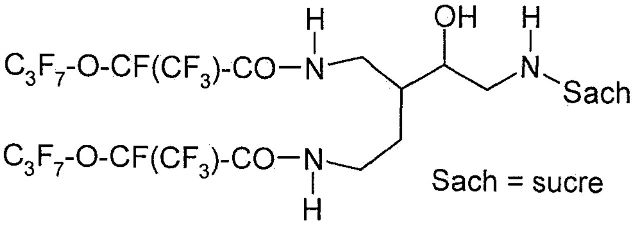

(III-8)

Sach = sucre

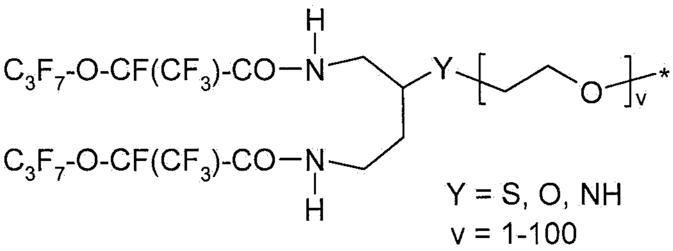

(III-9)

Y = S, O, NH

v = 1-100

**11.** Utilisation de composés selon l'une ou plusieurs parmi les revendications 1 à 10, comme additifs dans les peintures, les revêtements, les encres d'impression, les revêtements protecteurs, les revêtements spéciaux dans les applications électroniques ou optiques, les résines photosensibles, les revêtements antireflets supérieurs ou les revêtements antireflets inférieurs, les solutions de révélateur et les solutions de lavage ainsi que les résines photosensibles pour les procédés photo-lithographiques, les produits cosmétiques, les substances agrochimiques, les encaustiques, les revêtements photographiques ou les revêtements d'éléments optiques.

**12.** Composition comprenant des composés selon l'une ou plusieurs parmi les revendications 1 à 10, et un véhicule

qui est convenable pour l'application respective, et éventuellement d'autres substances actives spécifiques.

13. Composition selon la revendication 12, **caractérisée en ce que** la composition est constituée de préparations de peinture et de revêtement, de compositions d'extinction d'incendies, de lubrifiants, de compositions de lavage et de détergents, de dégivreurs, de solutions de révélateur et de solutions de lavage ainsi que de résines photosensibles pour les procédés photo-lithographiques, de produits cosmétiques, de substances agrochimiques, d'encaustiques ou de compositions d'hydrophobie pour la finition des textiles ou le traitement du verre.

Abbildung 1: statische Oberflächenspannung γ des Sulfosuccinates gemäß Beispiel 4

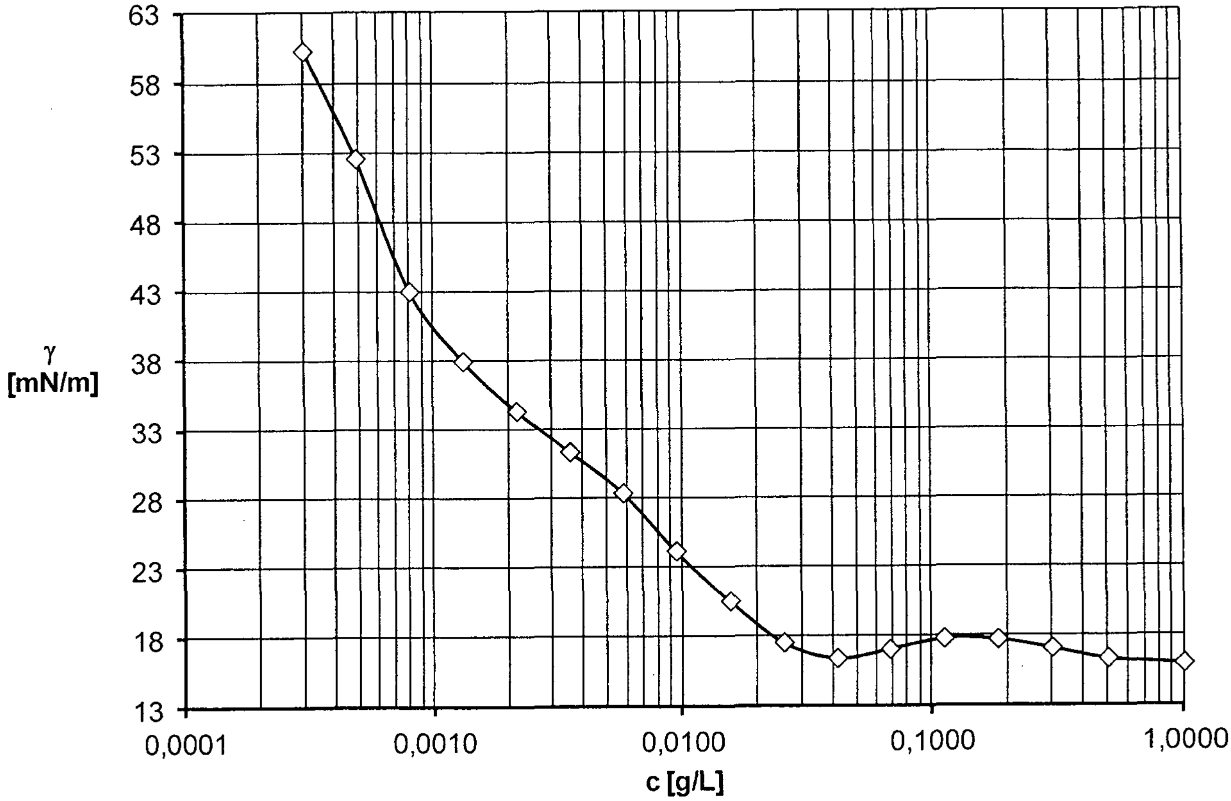

Abbildung 2: dynamischen Oberflächenspannungen γ des Sulfosuccinates gemäß Beispiel 4

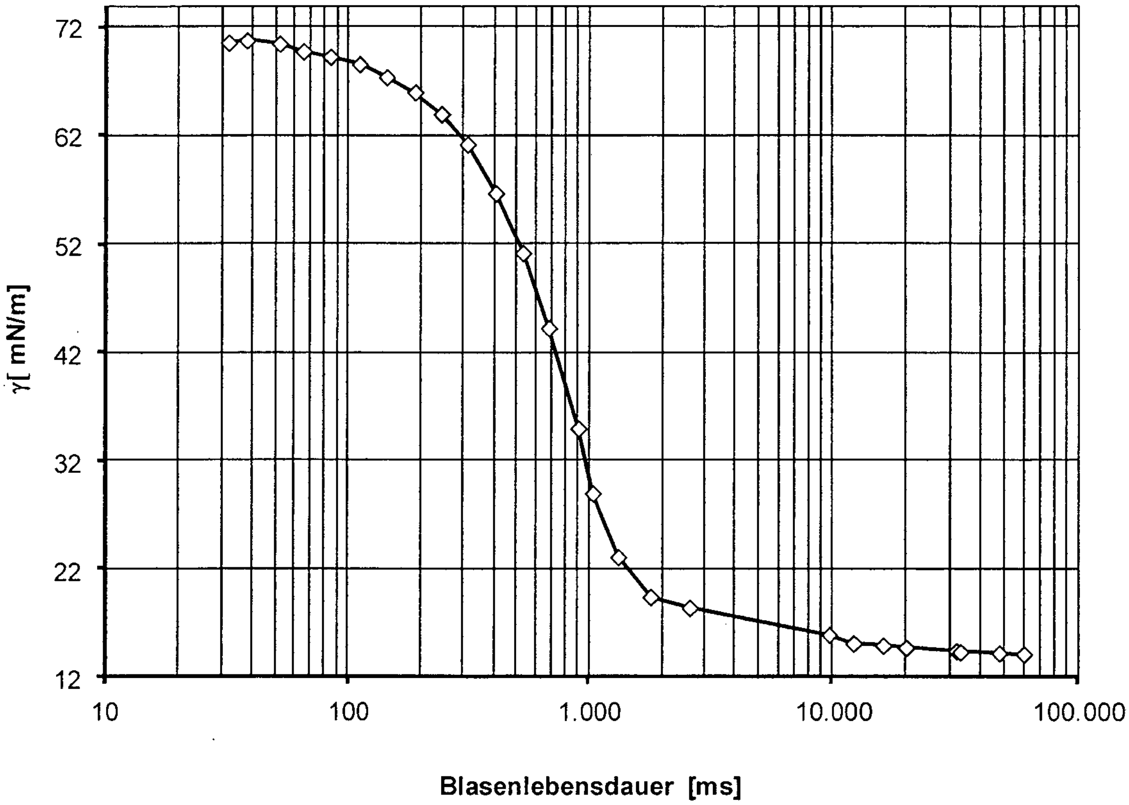

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 03010128 A
- JP 2001133984 A
- JP 9111286 A
- WO 2006072401 A
- WO 2010003567 A
- US 7635789 B
- US 20080093582 A
- JP 2004018394 A
- WO 2010002623 A
- EP 1762566 A1
- US 4968599 A
- US 4988610 A
- US 6890608 B
- WO 2009149807 A
- WO 2010149262 A
- WO 2011082770 A
- WO 2012084118 A


### In der Beschreibung aufgeführte Nicht-Patentliteratur


- *CHEMICAL ABSTRACTS,* 1313023-37-8
- *J. of Supercritical Fluids,* 2010, vol. 55, 802-816
- **A.R. PITT et al.** *Colloids and Surfaces A: Physicochemical and Engineering Aspects,* 1996, vol. 114, 321-335
- **A.R. PITT.** *Progr. Colloid Polym. Sci,* 1997, vol. 103, 307-317
- **Z.-T. LIU et al.** *Ind. Eng. Chem. Res.,* 2007, vol. 46, 22-28